(19) Europäisches Patentamt
European Patent Office
Office européen des brevets

(11) Veröffentlichungsnummer: **0 010 287**
**B1**

(12) # EUROPÄISCHE PATENTSCHRIFT

(45) Veröffentlichungstag der Patentschrift: **05.08.81**

(21) Anmeldenummer: **79103975.3**

(22) Anmeldetag: **15.10.79**

(51) Int. Cl.$^3$: **C 07 D 249/08,** C 07 D 249/04, C 07 D 233/60, C 07 D 409/06, C 07 D 405/06, A 01 N 43/50, A 01 N 43/64

(54) **Gamma-Azolylverbindungen, wachstumsregulierende Mittel, Verfahren zur Herstellung dieser und Verfahren zur Regulierung des Pflanzenwachstums.**

(30) Priorität: **18.10.78 DE 2845254**

(43) Veröffentlichungstag der Anmeldung: **30.04.80 Patentblatt 80/9**

(45) Bekanntmachung des Hinweises auf die Patenterteilung: **05.08.81 Patentblatt 81/31**

(84) Benannte Vertragsstaaten: **AT BE CH DE FR GB IT LU NL SE**

(56) Entgegenhaltungen:
**CH-A-568 712**
**DE-A-2 407 143**
**DE-A-547 953**
**DE-A-2 628 420**
**DE-A-2 633 492**
**DE-A-2 653 420**
**DE-A-2 708 987**
**DE-A-2 726 043**
**US-A-4 059 705**
**US-A-4 078 071**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Sauter, Hubert, Dr. Dipl.-Chem., Goethestrasse 23, D-6700 Ludwigshafen (DE)**
Erfinder: **Ammermann, Eberhard, Dr. Dipl.-Chem., Sachsenstrasse 3, D-6700 Ludwigshafen (DE)**
Erfinder: **Heilen, Gerd, Dr. Dipl.-Chem., Schifferstadter Strasse 1B, D-6720 Speyer (DE)**
Erfinder: **Jung, Johann, Dr. Dipl.-Landwirt, Hardenburgstrasse 19, D-6703 Limburgerhof (DE)**

EP 0 010 287 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Gamma-Azolylverbindungen, wachstumsregulierende Mittel, Verfahren zur Herstellung dieser und Verfahren zur Regulierung des Pflanzenwachstums

Die vorliegende Erfindung betrifft neue wertvolle γ-Azolyl-Verbindungen, insbesondere Ether, Ester und Carbamate, Verfahren zu ihrer Herstellung, Mittel zur Beeinflussung des Pflanzenwachstums, die diese Verbindungen als Wirkstoffe enthalten, und Verfahren zur Regulierung des Pflanzenwachstums mit diesen Verbindungen.

Es ist bekannt, β-Triazolylether wie 1-(4′-Bromphenyl)-1-allyloxy-2-(1″,2″,4″-triazolyl-(1″))-ethan zur Beeinflussung des Pflanzenwachstums von Raps, Weizen, Hafer, Roggen und Gerste zu verwenden (Deutsche Offenlegungsschrift 2650831). Deren Wirkung ist jedoch, vor allem bei niedrigen Anwendungskonzentrationen, nicht immer ausreichend.

Ferner ist es bekannt, dass das 2-Chlorethyltrimethylammoniumchlorid (Chlorcholinchlorid, CCC) (US-Patentschrift 3156544) pflanzenwuchsregulierende Eigenschaften besitzt. Mit seiner Hilfe lässt sich z.B. eine Hemmung des Längenwachstums bei einigen Getreidearten und eine Hemmung des vegetativen Wachstums bei einigen wenigen Kulturpflanzen erreichen. Die Wirkung dieses Stoffes ist jedoch, insbesondere bei niedrigen Aufwandmengen, nicht immer befriedigend.

Es wurde nun gefunden, dass Verbindungen der Formel

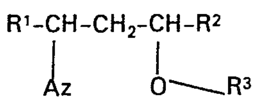

I

in der $R^1$ und $R^2$ verschieden oder gleich sind und Alkyl mit 1 bis 8 C-Atomen, Furanyl, Thienyl, Naphthyl oder Phenyl bedeuten, wobei der Phenylrest durch Fluor, Chlor, Brom, Nitro, Trifluormethyl, ferner durch Alkyl, Alkoxy oder Alkenyl mit 1 bis 4 C-Atomen substituiert sein kann und $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl, ferner durch Alkyl oder Alkoxy mit 1 bis 4 C-Atomen substituiert sein kann oder $R^3$ einen $-CO-R^4$ Rest bedeutet, wobei $R^4$ einen gegebenenfalls durch Halogen, Alkoxy, Oxo (= O) oder Carboxyalkyl substituierten Alkylrest mit 1 bis 5 C-Atomen oder einen Phenylrest bedeutet und $R^3$ ferner einen $-CO-NH-R^5$ Rest bedeutet, wobei $R^5$ einen Alkylrest mit 1 bis 4 C-Atomen oder einen Chlorphenylrest bedeutet und
Az einen über ein Ringstickstoffatom gebundenen Imidazolyl-, 1,2,4-Triazolyl- oder einen 1,2,3-Triazolylrest bedeutet, eine ausgezeichnete Wirkung bei der Beeinflussung des Pflanzenwachstums haben und eine sehr gute Pflanzenverträglichkeit besitzen.

$R^1$ und $R^2$ bedeuten beispielsweise Methyl, n-Propyl, Isopropyl, n-Butyl, sek.-Butyl, iso-Butyl, tert.-Butyl, 3-Pentyl, Neopentyl, 2-Furanyl, 2-Thiophenyl, 4-Fluorphenyl, 2- und 4-Chlorphenyl, 2,4-Dichlorphenyl, 4-Bromphenyl, p-Tolyl, 2- und 4-Methoxyphenyl, 4-Isopropylphenyl und 1-Naphthyl.

$R^3$ bedeutet beispielsweise Methyl, Ethyl, n-Propyl, Allyl, Propargyl, n-Butyl, 1-Crotyl, n-Pentyl, 3-Methyl-2-buten-1-yl, n-Hexyl, n-Octyl, 1-(Carbomethoxy)-1-ethyl, Benzyl, 4-Fluorbenzyl, 4-Chlorbenzyl, 2,4-Dichlorbenzyl, 3- und 4-Brombenzyl, 4-Methylbenzyl, 4-tert.-Butylphenyl und 4-Metoxybenzyl.

$-CO-R^4$ bedeutet beispielsweise Acetyl, Chloracetyl, Propionyl, Butyryl, Acetoacetyl, Pivaloyl und Benzoyl.

$-CO-NH-R^5$ bedeutet beispielsweise Methylcarbamoyl und 4-Chlorphenylcarbamoyl. Az bedeutet z.B. 1-Imidazolyl und vorzugswseise 1,2,4-Triazol-1-yl.

Weiterhin wurde gefunden, dass man γ-Azolylverbindungen der Formel I erhält, wenn man γ-Azolylalkoholate der Formel

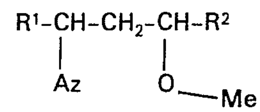

II

in welcher $R^1$, $R^2$ und Az die oben angegebene Bedeutung haben, und Me Natrium oder Kalium bedeutet, umsetzt mit Organohalogeniden der Formel

$R^3$–X III

in der $R^3$ die oben angegebene Bedeutung hat, und X vorzugsweise Chlor oder Brom bedeutet, aber auch Jod bedeuten kann. Diese Umsetzungen werden beispielsweise in einem indifferenten Verdünnungsmittel, wie Ethylether, Tetrahydrofuran, Dioxan, Toluol, Xylol, Dimethylformamid bei Temperaturen von etwa 0 bis 100 °C, vorzugsweise +30 bis +50 °C durchgeführt.

Die γ-Azolylalkoholate der Formel II gewinnt man bequem aus den γ-Azolylalkanolen der allgemeinen Formel

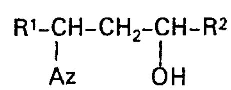

IV

in der $R^1$, $R^2$ und Az die oben genannten Bedeutungen haben, mit starken Basen wie Alkalihydrid, Alkaliamid oder niederen Alkalialkoholaten, wobei in letzterem Falle die entstehenden niederen Alkohole durch azeotrope Destillation entfernt werden müssen. Dafür eignen sich besonders gut molare Mengen oder bis zu 50%ige Überschüsse an Lithiumhydrid, Natriumhydrid, Natriumamid, Natriummethoxid, Natriumethoxid oder Kalium-tert.-butoxid. Eine Isolierung der Alkalisalze II erübrigt sich im allgemeinen. Sie können in einem inerten Lösungsmtitel z.B. Toluol, Xylol, Tetrahydrofuran, Dioxan oder Dimethylformamid sofort nach ihrer Herstellung mit den Verbindungen der Formel III weiter umgesetzt werden. Die Verwendung von Phasentransfer-Katalysatoren wie beispielsweise Benzyl-triethylammoniumchlorid, Tetrabutylammonium-methosulfat oder Tetrapentylphosphoniumbromid erlaubt die Verwendung von wässrigen Laugen wie beispielsweise 50%ige Natronlauge. Dabei werden die Verbindungen der Formel III in 10 bis 20 molarem Überschuss verwendet, wobei sie auch als Verdünnungsmittel fungieren. Diese Umsetzungen werden bei Temperaturen von etwa 0 bis 90 °C, vorzugsweise +10 bis +50 °C durchgeführt.

Man erhält ferner die erfindungsgemässen Verbindungen der Formel I, wenn man γ-Azolylalkohole der Formel IV mit Säurechloriden oder Säureanhydriden der Formel

$R^4$-CO-Y V

in der $R^4$ die oben angegebene Bedeutung hat und Y Chlor, Brom oder den Rest -O-CO-$R^4$ bedeutet,
oder mit Isocyanaten der Formel

$R^5$-NH-COCl VI

oder mit Carbamoylchloriden der Formel

$R^5$-NH-COCl VII

in welcher $R^5$ die oben angegebene Bedeutung hat, gegebenenfalls in Gegenwart eines basischen Katalysators oder eines säurebindenden Mittels und gegebenenfalls in Gegenwart eines Verdünnungsmittels umsetzt.

Die als Ausgangsmaterial zu verwendenden γ-Azolylalkohole sind durch die Formel IV allgemein definiert. Man erhält sie beispielsweise, indem man bekannte β-Azolylketone (vgl. Deutsche Offenlegungsschriften 2634511 und 2656728) der Formel

$R^1$-CH-$CH_2$-C-$R^2$ VIII
|    ||
Az   O

in welcher $R^1$, $R^2$ und Az die oben angegebene Bedeutung haben, reduziert. Die Reduktion kann zum Beispiel durchgeführt werden:

a) mit komplexen Hydriden, wie Natriumborhydrid in Gegenwart eines polaren Lösungsmittels, wie zum Beispiel Methanol, oder mit Lithiumalanat in Diethylether oder Tetrahydrofuran oder Dioxan, bei Temperaturen zwischen 0 °C und 30 °C oder

b) mit Wasserstoff in Gegenwart eines Katalysators, wie Platin oder Raney-Nickel und in Gegenwart eines polaren Lösungsmittels, wie Methanol, Ethanol, Isopropanol, bei einer Temperatur zwischen 40 und 60 °C und einem Druck zwischen 1 bis 100 bar, oder

c) mit Aluminiumisopropylat in Gegenwart eines inerten Lösungsmittels z.B. Isopropanol bei einer Temperatur zwischen 40 und 120 °C und anschliessender Hydrolyse, z.B. mit wässriger Salzsäure.

Dabei entstehen Diastereomerengemische, da die Verbindungen der allgemeinen Formel IV zwei chirale Kohlenstoffatome besitzen. Sie können deshalb in der erythro- sowie in der threo-Form vorliegen. In beiden Fällen liegen sie als Racemate vor. Die erythro- und threo-Formen lassen sich bei einigen der γ-Azolylalkohole und bei einigen der daraus hergestellten, erfindungsgemässen Verbindungen der Formel I beispielsweise durch Löslichkeitsunterschiede oder durch Säulenchromatographie trennen und in reiner Form isolieren.

Die für die Herstellung der erfindungsgemässen Wirkstoffe erforderlichen Organohalogenide der Formel III oder die Säurechloride und Säureanhydride der Formel V oder Isocyanate der Formel VI und die Carbamoylchloride der Formel VII sind bekannt oder lassen sich nach üblichen Verfahren herstellen. Diese Methoden sind aus den allgemeinen Lehrbüchern der organischen Chemie bekannt.

Die Umsetzungen der γ-Azolylalkohole der Formel IV mit Ausgangsstoffen der Formel V, VI und VII werden zweckmässig ohne Verdünnungsmittel oder in einem inerten Lösungsmittel durchgeführt. Hierzu gehören Acetonitril, Ether wie Diethyl-, Dipropyl-, Dibutylether, Tetrahydrofuran, Dioxan und Dimethoxyethan; Ester wie Essigsäureethylester; Ketone, wie Aceton und Methylethyl-keton; chlorierte Kohlenwasserstoffe, wie Methylenchlorid, Chloroform, Tetrachlorkohlenstoff, Dichlorethan, Tetrachlorethan, Chlorbenzol und Kohlenwasserstoffe, wie Benzol, Toluol und Xylol.

Dabei ist es zweckmässig, eine Base wie beispielsweise Natriumhydroxid, Natriumcarbonat, Kaliumcarbonat, Calciumcarbonat, Natriumacetat; Amine, wie Triethylamin, Pyridin, Imidazol oder Piperidin als Hilfsbasen oder als Katalysatoren zuzusetzen. Die Reaktionstemperatur kann dabei zwischen -25 bis +100 °C, vorzugsweise zwischen +10 und +80 °C variiert werden.

Die so erhaltenen Verbindungen der Formel I werden nach üblichen Methoden isoliert und gegebenenfalls gereinigt.

Als Beispiele für die neuen γ-Azolyl-Verbindungen seien im einzelnen genannt:
2-Methoxy-4-(1,2,4-triazolyl-(1))-5,5-dimethylhexan,
2-n-Propoxy-4-(1,2,4-triazolyl-(1))-5,5-dimethylhexan,
2-Allyloxy-4-(1,2,4-triazolyl-(1))-5,5-dimethylhexan,
2-n-Butoxy-4-(1,2,4-triazolyl-(1))-5,5-dimethylhexan,
2-n-Pentyloxy-4-(1,2,4-triazolyl-(1))-5,5-dimethylhexan,
2-n-Octyloxy-4-(1,2,4-triazolyl-(1))-5,5-dimethylhexan,
2-Benzyloxy-4-(1,2,4-triazolyl-(1))-5,5-dimethylhexan,
2-Pivaloyloxy-4-(1,2,4-triazolyl-(1))-5,5-dimethylhexan,
2,2-Dimethyl-3-(1,2,4-triazolyl-(1))-5-allyloxy-nonan,

2,2,4,4-Tetramethyl-3-(1,2,4-triazolyl-(1))-5-allyloxy-heptan,
1-Methoxy-1-phenyl-3-(1,2,4-triazolyl-(1))-4,4-dimethylpentan,
1-Allyloxy-1-phenyl-3-(1,2,4-triazolyl-(1))-4,4-dimethylpentan,
1-Methoxy-1-phenyl-3-(1,2,4-triazolyl-(1))-5-methylhexan,
1-Phenyl-1-(1,2,4-triazolyl-(1))-3-ethoxy-butan,
1-Phenyl-1-(1,2,4-triazolyl-(1))-3-allyloxy-butan,
1-Phenyl-1-(1,2,4-triazolyl-(1))-3-(1-butoxy)-butan,
1-Phenyl-1-(1,2,4-triazolyl-(1))-3-(1-hexyloxy)-butan,
1-Phenyl-1-(1,2,4-triazolyl-(1))-3-(1-octyloxy)-butan,
1-Phenyl-1-(1,2,4-triazolyl-(1))-3-benzyloxy)-butan,
1-Phenyl-1-(1,2,4-triazolyl-(1))-3-(4-chlorbenzyloxy)-butan,
1-Phenyl-1-(1,2,4-triazolyl-(1))-3-pivaloyloxy-butan
1-(4-Chlorbenzyloxy)-1-phenyl-3-(1,2,4-triazolyl-(1))-4,4-dimethylpentan,
1-Methoxy-1-(4-fluorphenyl)-3-(1,2,4-triazolyl-(1))-4,4-dimethylpentan,
1-Allyloxy-1-(4-fluorphenyl)-3-(1,2,4-triazolyl-(1))-4,4-dimethylpentan,
1-Benzyloxy-1-(4-fluorphenyl)-3-(1,2,4-triazolyl-(1))-4,4-dimethylpentan,
1-(4-Chlorbenzyloxy)-1-(4-fluorphenyl)-3-(1,2,4-triazolyl-(1))-4,4-dimethylpentan,
1-(4-Methoxy-benzyloxy)-1-(4-fluorphenyl)-3-(1,2,4-triazolyl-(1))-4,4-dimethylpentan,
1-Acetoxy-1-(4-fluorphenyl)-3-(1,2,4-triazolyl-(1))-4,4-dimethylpentan,
1-(2-Chlorphenyl)-1-(1,2,4-triazolyl-(1))-3-allyloxy-butan,
1-(2-Chlorphenyl)-1-(1,2,4-triazolyl-(1))-3-(1-propoxy)-butan,
1-(2-Chlorphenyl)-1-(1,2,4-triazolyl-(1))-3-(1-butoxy)-butan,
1-(2-Chlorphenyl)-1-(1,2,4-triazolyl-(1))-3-(1-hexyloxy)-butan,
1-(2-Chlorphenyl)-1-(1,2,4-triazolyl-(1))-3-allyloxy-4,4-dimethyl-pentan,
1-(4-Chlorphenyl)-1-(1,2,4-triazolyl-(1))-3-ethoxy-butan,
1-(4-Chlorphenyl)-1-(1,2,4-triazolyl-(1))-3-allyloxy-butan,
1-(4-Chlorphenyl)-1-(1,2,4-triazolyl-(1))-3-(1-pentyloxy)-butan,
1-(4-Chlorphenyl)-1-(1,2,4-triazolyl-(1))-3-(4-chlorbenzyloxy)-butan,
1-(4-Chlorphenyl)-1-(1,2,4-triazolyl-(1))-3-(2,4-dichlorbenzyloxy)-butan,
1-Methoxy-1-(4-chlorphenyl)-3-(1,2,4-triazolyl-(1))-4,4-dimethylpentan,
1-Allyloxy-1-(4-chlorphenyl)-3-(1,2,4-triazolyl-(1))-4,4-dimethylpentan,
1-Allyloxy-1-(4-chlorphenyl)-3-(1,2,4-triazolyl-(1))-5-methylhexan,
1-Allyloxy-1-(4-chlorphenyl)-3-(1,2,4-triazolyl-(1))-5-ethylhexan,
1-Crotyloxy-1-(4-chlorphenyl)-3-(1,2,4-triazolyl-(1))-4,4-dimethylpentan,
1-(3-Methyl-2-buten-1-yloxy)-1-(4-chlorphenyl)-3-(1,2,4-triazolyl-(1))-4,4-dimethylpentan,
1-Propargyloxy-1-(4-chlorphenyl)-3-(1,2,4-triazolyl-(1))-4,4-dimethylpentan,
2-(1-(4-Chlorphenyl)-3-(1,2,4-triazolyl-(1))-4,4-dimethyl-pentan-1-oxy)-propionsäure-methylester,
1-Benzyloxy-1-(4-chlorphenyl)-3-(1,2,4-triazolyl-(1))-4,4-dimethylpentan,
1-(4-Fluorbenzyloxy)-1-(4-chlorphenyl)-3-(1,2,4-triazolyl-(1))-4,4-dimethylpentan,
1-(4-Chlorbenzyloxy)-1-(4-chlorphenyl)-3-(1,2,4-triazolyl-(1))-4,4-dimethylpentan,
1-(4-Brombenzyloxy)-1-(4-chlorphenyl)-3-(1,2,4-triazolyl-(1))-4,4-dimethylpentan,
1-(4-Methylbenzyloxy)-1-(4-chlorphenyl)-3-(1,2,4-triazolyl-(1))-4,4-dimethylpentan,
1-(3-Trifluormethyl-benzyloxy)-1-(4-chlorphenyl)-3-(1,2,4-triazolyl-(1))-4,4-dimethylpentan,
1-(4-tert.-Butyl-benzyloxy)-1-(4-chlorphenyl)-3-(1,2,4-triazolyl-(1))-4,4-dimethylpentan,
1(2,4-Dichlorbenzyloxy)-1-(4-chlorphenyl)-3-(1,2,4-triazolyl-(1))-4,4-dimethylpentan,
1-Acetoxy-1-(4-chlorphenyl)-3-(1,2,4-triazolyl-(1))-4,4-dimethylpentan,
1-(4-Chlorphenyl-1-(1,2,4-triazolyl-(1))-3-acetoxy-4,4-dimethylpentan,
1-Propionyloxy-1-(4-chlorphenyl)-3-(1,2,4-triazolyl-(1))-4,4-dimethylpentan,
1-Acetylacetoxy-1-(4-chlorphenyl)-3-(1,2,4-triazolyl-(1))-4,4-dimethylpentan,
1-(Methylcarbamoyl)-1-(4-chlorphenyl)-3-(1,2,4-triazolyl-(1))-4,4-dimethylpentan,
1-(4-Chlorphenylcarbamoyl)-1-(4-chlorphenyl)-3-(1,2,4-triazolyl-(1))-4,4-dimethylpentan,
1-Methoxy-1-(4-bromphenyl)-3-(1,2,4-triazolyl-(1))-4,4-dimethylpentan,
1-Allyloxy-1-(4-bromphenyl)-3-(1,2,4-triazolyl-(1))-4,4-dimethylpentan,
1-(Methoxy-1-(4-tolyl)-3-(1,2,4-triazolyl-(1))-4,4-dimethylpentan,
1-Allyloxy-1-(4-tolyl)-3-(1,2,4-triazolyl-(1))-4,4-dimethylpentan,
1-Methoxy-1-(4-methoxyphenyl)-3-(1,2,4-triazolyl-(1))-4,4-dimethylpentan,
1-n-Propoxy-1-(4-methoxyphenyl)-3-(1,2,4-triazolyl-(1))-4,4-dimethylpentan,
1-Acetoxy-1-(4-methoxyphenyl)-3-(1,2,4-triazolyl-(1))-4,4-dimethylpentan,
1-(2-Methoxyphenyl)-1-(1,2,4-triazolyl-(1))-3-ethoxy-butan,
1-(2-Methoxyphenyl)-1-(1,2,4-triazolyl-(1))-3-allyloxy-butan,
1-(2-Methoxyphenyl)-1-(1,2,4-triazolyl-(1))-3-(1-butoxy)-butan,
1-(2-Methoxyphenyl)-1-(1,2,4-triazolyl-(1))-3-(1-hexyloxy)-butan,
1-(2-Methoxyphenyl)-1-(1,2,4-triazolyl-(1))-3-(1-octyloxy)-butan,
1-(2-Methoxyphenyl)-1-(1,2,4-triazolyl-(1))-3-benzyloxy-butan,
1-(2-Methoxyphenyl)-1-(1,2,4-triazolyl-(1))-3-(4-chlorbenzyloxy)-butan,

1-(2-Methoxyphenyl)-1-(1,2,4-triazolyl-(1))-3-pivaloyloxy-butan,
1-(2-Methoxyphenyl)-1-(1,2,4-triazolyl-(1))-3-allyloxy-4,4-dimethylpentan,
1-(2-Methoxyphenyl)-1-(1,2,4-triazolyl-(1))-3-(1-butoxy)-4,4-dimethylpentan,
1-(2-Methoxyphenyl)-1-(1,2,4-triazolyl-(1))-3-acetoxy-4,4-dimethylpentan,
1-(2-Methoxyphenyl)-1-(1,2,4-triazolyl-(1))-3-chloracetoxy-4,4-dimethylpentan,
1-Methoxy-1-(2,4-dichlorphenyl)-3-(1,2,4-triazolyl-(1))-4,4-dimethylpentan,
1-Allyloxy-1-(2,4-dichlorphenyl)-3-(1,2,4-triazolyl-(1))-4,4-dimethylpentan,
1-Propargyloxy-1-(2,4-dichlorphenyl)-3-(1,2,4-triazolyl-(1))-4,4-dimethylpentan,
1-Benzyloxy-1-(2,4-dichlorphenyl)-3-(1,2,4-triazolyl-(1))-4,4-dimethylpentan,
1-(2,4-Dichlorphenyl)-1-(1,2,4-triazolyl-(1))-3-allyloxy-4,4-dimethylpentan,
1-(2,4-Dichlorphenyl)-1-imidazolyl-3-acetoxy-4,4-dimethylpentan,
1-(4-Isopropyl-phenyl)-1-(1,2,4-triazolyl-(1))-3-(methylcarbamoyl)-4,4-dimethylpentan,
1-Methoxy-1-(1-naphthyl)-3-(1,2,4-triazolyl-(1))-4,4-dimethylpentan,
1-Allyloxy-1-(1-naphthyl)-3-(1,2,4-triazolyl-(1))-4,4-dimethylpentan,
1-(1-Naphthyl)-1-(1,2,4-triazolyl-(1))-3-methoxy-butan,
1-(1-Naphthyl)-1-(1,2,4-triazolyl-(1))-3-(1-butoxy)-butan,
1-(1-Naphthyl)-1-(1,2,4-triazolyl-(1))-3-benzyloxy-butan,
1-(1-Naphthyl)-1-(1,2,4-triazolyl-(1))-3-allyloxy-4,4-dimethylpentan,
1-Methoxy-1-(2-furanyl)-3-(1,2,4-triazolyl-(1))-4,4-dimethylpentan,
1-Methoxy-1-(2-thiophenyl)-3-(1,2,4-triazolyl-(1))-4,4-dimethylpentan,
1-Allyloxy-1-(2-thiophenyl)-3-(1,2,4-triazolyl-(1))-4,4-dimethylpentan,
1-(4-Chlorbenzyloxy)-1-(2-thiophenyl)-3-(1,2,4-triazolyl-(1))-4,4-dimethylpentan,
1-Methoxy-1,3-diphenyl-3-(1,2,4-triazolyl-(1))-propan,
1-Allyloxy-1,3-diphenyl-3-(1,2,4-triazolyl-(1))-propan,
1-Benzyloxy-1,3-diphenyl-3-(1,2,4-triazolyl-(1))-propan,
1-(4-Chlorbenzyloxy)-1,3-diphenyl-3-(1,2,4-triazolyl-(1))-propan.

Die neuen Verbindungen können praktisch alle Entwicklungsstadien einer Pflanze verschiedenartig beeinflussen. Sie werden deshalb als Pflanzenwachstumsregulatoren verwendet.

Die Wirkungsvielfalt der Pflanzenwachstumsregulatoren hängt ab vor allem
a) von der Pflanzenart und -sorte,
b) von dem Zeitpunkt der Applikation, bezogen auf das Entwicklungsstadium der Pflanzen und auf die Jahreszeit,
c) von dem Applikationsort und -verfahren (Samenbeize, Bodenbehandlung oder Blattapplikation),
d) von den geoklimatischen Faktoren (Temperatur, Niederschlag),
e) von der Bodenbeschaffenheit (einschliesslich Düngung)
f) von der Formulierung bzw. Anwendungsform des Wirkstoffes und schliesslich
g) von den angewendeten Konzentrationen der aktiven Substanz.

Von den verschiedenartigen Anwendungsmöglichkeiten der Pflanzenwachstumsregulatoren in der Landwirtschaft und im Gartenbau werden einige nachstehend erwähnt.

A. Mit den erfindungsgemäss verwendbaren Verbindungen lässt sich das vegetative Wachstum der Pflanzen stark hemmen, was sich insbesondere in einer Reduzierung des Längenwachstums äussert. Die behandelten Pflanzen weisen demgemäss einen gedrungenen Habitus auf; ausserdem ist eine dunklere Blattfärbung zu beobachten.

Als vorteilhaft für die Praxis erweist sich z.B. die Verringerung des Grasbewuchses an Strassenrändern, Kanalböschungen und auf Rasenflächen wie Park-, Sport- und Obstanlagen, Zierrasen und Flugplätzen, so dass die arbeits- und kostenaufwendige Schnittarbeit reduziert werden kann.

Von wirtschaftlichem Interesse ist auch die Erhöhung der Standfestigkeit von lageranfälligen Kulturen wie Getreide, Mais und Soja. Die dabei verursachte Halmverkürzung und Halmverstärkung verringern oder beseitigen die Gefahr des «Lagerns» (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung der Reife bei Baumwolle.

Damit wird ein vollständig mechanisiertes Ernten dieser wichtigen Kulturpflanzen ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumshemmern lässt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide, ist es vorteilhaft, wenn die Bestände durch Behandlung mit erfindungsgemässen Verbindungen im Herbst zwar gut bestockt werden, aber nicht zu üppig in den Winter hineingehen. Dadurch kann der erhöhten Frostempfindlichkeit und – wegen der relativ geringen Blatt- bzw. Pflanzenmasse – dem Befall mit verschiedenen Krankheiten (z.B. Pilzkrankheit) vorgebeugt werden. Die Hemmung des vegetativen Wachstums ermöglicht ausserdem bei vielen Kulturpflanzen eine dichtere Bepflanzung des Bodens, so dass ein Mehrertrag bezogen auf die Bodenfläche erzielt werden kann.

B. Mit den erfindungsgemäss verwendbaren Wirkstoffen lassen sich Mehrerträge sowohl an Pflanzenteilen als auch an Pflanzeninhaltsstoffen erzielen. So ist es beispielsweise möglich, höhere Produktionen an Knospen, Blüten, Blättern, Früchten, Samenkörnern, Wurzeln und Knollen zu induzieren, den Gehalt an Zucker in Zuckerrüben, Zuckerrohr, sowie Zitrusfrüchten zu erhöhen, den Proteingehalt in Getreide oder Soja zu steigern oder Gummibäume zu vermehrtem Latexfluss zu stimulieren.

Dabei können die erfindungsgemässen Stoffe Ertragssteigerungen durch Eingriffe in den pflanzlichen Stoffwechsel bzw. durch Förderung oder Hemmung des vegetativen und/oder des generativen Wachstums hervorrufen.

C. Mit Pflanzenwachstumsregulatoren lassen sich schliesslich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die eine chemische, zeitlich konzentrierte Lockerung der Haftfestigkeit der Stiele an den Zweigen (Abszission) von Zitrusfrüchten, von Oliven oder von anderen Arten und Sorten von Kern-, Stein- und Schalenobst ermöglichen kann. Derselbe Mechanismus, das heisst die Förderung der Ausbildung von Trenngewebe zwischen Frucht- bzw. Blatt und Sprossteil der Pflanze ist auch für ein chemisch induziertes, gut kontrollierbares Entblättern der Pflanzen verantwortlich.

Die Wirkung der erfindungsgemässen Verbindungen ist besser als bei bekannten Wachstumregulatoren. Die Wirkung zeigt sich sowohl bei Monokotylen, z.B. Getreide wie Weizen, Gerste, Roggen, Hafer und Reis oder Mais als auch bei Dikotylen (z.B. Sonnenblumen, Tomaten, Soja, Reben, Baumwolle und vor allem Raps) und verschiedenen Zierpflanzen wie Chrysanthemen, Poinsettien und Hibiskus.

Die erfindungsgemässen Verbindungen können den Kulturpflanzen sowohl durch den Samen (als Saatgutbeizmittel), als auch über den Boden, d.h. durch die Wurzel sowie durch Spritzung über das Blatt zugeführt werden. Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,1 bis 12 kg/ha bevorzugt, 0,25 bis 3 kg/ha als ausreichend zu betrachten.

Die erfindungsgemässen Mittel können in üblichen Formulierungen angewendet werden, wie Lösungen, Emulsionen, Suspensionen, Stäube, Pulver, Pasten oder Granulate. Die Anwendungsformen richten sich ganz nach den Verwendungszwecken; sie sollen in jedem Fall eine feine und gleichmässige Verteilung der wirksamen Substanzen gewährleisten. Die Formulierungen werden in bekannter Weise hergestellt, z.B. durch Vermischen des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene Emulgatoren (z.B. Polyoxyethylen-Fettalkohol-Ether, Alkylsulfonate) und Dispergiermittel, wie Lignin, Sulfitablaugen und Methylcellulose.

Die Formulierungen enthalten im allgemeinen zwischen 0,1 und 95 Gewichtsprozent Wirkstoff, vorzugsweise zwischen 0,5 und 90%.

Die Formulierungen, bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Die erfindungsgemässen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrösserung des Wirkungsspektrums. Bei einer Anzahl solcher Wachstumregulatormischungen treten auch synergistische Effekte auf, d.h. die Wirksamkeit des Kombinationsproduktes ist grösser als die addierten Wirksamkeiten der Einzelkomponenten.

Die folgenden Beispiele erläutern die Herstellung der neuen Verbindungen:

Beispiel 1

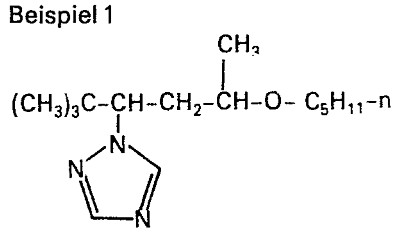

1a) Unter kräftigem Rühren wird eine Mischung aus 9,2 g 5,5-Dimethyl-4-(1,2,4-triazolyl-(1))-hexan-2-ol, 50 g 1-Chlorpentan, 2 g Tetrabutylammoniumhydrogensulfat und 32 g 50%iger wässriger Natriumhydroxidlösung 20 Stunden auf 50 °C erwärmt. Die Mischung wird sodann mit 200 ml Wasser versetzt und zweimal mit je 100 ml Methylenchlorid extrahiert. Die vereinigten Extrakte werden achtmal mit je 100 ml Wasser ausgeschüttelt, über Magnesiumsulfat getrocknet und im Vakuum – schliesslich bis 50 °C und 0,1 Torr – eingeengt.

Man erhält 10,5 g (79% d. Theorie) 2-(1-Pentyloxy)-4-(1,2,4-triazolyl-(1))-5,5-dimethylhexan als blassgelbes, öliges Diastereomerengemisch.

$^1$H-NMR (80 MHz/$CDCl_3$):δ=0,7–1,5 (m, 19H, darin enthalten: s bei 0,9, 9H), 1.5–4.6 (mehrere m, 7H), 3.9–4.4 (2dd, zus. 1H) 7.8–8.1 ppm (4s zus. 2H).

Herstellung des Ausgangsproduktes:

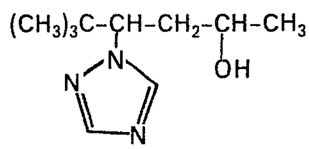

1b) Eine Lösung von 55 g 5,5-Dimethyl-3-hexen-2-on (K.N. Campbell, J. Amer. Chem. Soc., 59, 1980 (1937)), 60 g 1,2,4-Triazol und 5 g Kaliumhydroxid in 300 ml Ethanol wird zwei Stunden am Rückfluss erhitzt. Nach Abdestillieren des Lösungsmittels im Vakuum wird der Rückstand in 300 ml Methylenchlorid aufgenommen, dreimal mit je 100 ml Wasser gewaschen, über Magnesiumsulfat getrocknet und eingeengt. Der Rückstand wird bei 0 °C mit 100 ml Diisopropylether gerührt und der weisse, kristalline Niederschlag agesaugt und getrocknet.

Man erhält 83 g (97% d. Theorie) 5,5-Dimethyl-4-(1,2,4-triazolyl-(1))-hexan-2-on vom Schmelzpunkt 75 bis 76 °C.

1c) Zu einer Lösung von 54 g 5,5-Dimethyl-4-(1,2,4-triazolyl-(1))-hexan-2-on in 500 ml Ethanol werden bei 20 °C unter gutem Rühren 13,5 g Natriumborhydrid portionsweise zugegeben. Nach 17stündigem Rühren bei 20 °C wird das Gemisch eingeengt. Der Rückstand wird mit 270 ml 20%iger Kalilauge 1 Stunde gerührt und mit 500 ml Ether extrahiert. Die Etherphase wird über Magnesiumsulfat getrocknet und eingeengt. Das zurückbleibende farblose Öl kristallisiert nach dreitägigem Stehen vollständig durch. Man isoliert 44,8 g (83% der Theorie) 5,5-Dimethyl-4-(1,2,4-triazolyl-(1))-hexan-2-ol als weisse Kristalle vom Schmelzpunkt 73-75 °C.

Beispiel 2

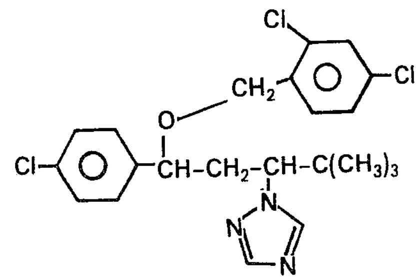

2a) Einer Suspension von 2,4 g Natriumhydrid in 100 ml trockenem Tetrahydrofuran wird die Lösung von 20,5 g 1-(4-Chlorphenyl)-3-(1,2,4-triazolyl-(1))-4,4-dimethylpentan-1-ol in 100 ml Tetrahydrofuran zugetropft. Nach 24stündigem Rühren bei Raumtemperatur wird das Gemisch mit der Lösung von 15,7 g 2,4-Dichlorbenzylchlorid in 50 ml Tetrahydrofuran tropfenweise versetzt.

Nach 48stündigem Rühren wird das Reaktionsgemisch vorsichtig mit 30 ml Wasser zersetzt und eingeengt. Der Rückstand wird in 300 ml Ether aufgenommen, dreimal mit je 100 ml Wasser gewaschen, die Etherphase getrocknet und eingeengt. Der Rückstand wird mit 25 ml Petrolether und 25 ml Ether über Nacht bei 0 °C gelassen. Der kristalline, farblose Niederschlag wird abgesaugt, mit Petrolether gewaschen und getrocknet.

Man erhält 24,9 g (79% der Theorie) 1-(2,4-Dichlorbenzyloxy)-1-(4-chlorphenyl)-3-(1,2,4-triazolyl-(1))-4,4-dimethylpentan vom Schmelzpunkt 104 bis 107 °C.

Herstellung des Ausgangsproduktes

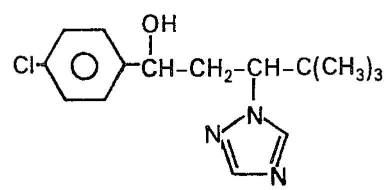

2b) Analog Beispiel 1c) wurde das 1-(4-Chlorphenyl)-3-(1,2,4-triazolyl-(1))-4,4-dimethylpentan-1-ol als glasklares Harz in 81%iger Ausbeute aus dem entsprechenden β-Triazolylketon durch Reduktion mit Natriumborhydrid hergestellt.

Das aus 1-(4-Chlorphenyl)-4,4-dimethyl-2-pentan-1-on durch Umsetzung mit 1,2,4-Triazol in 86%iger Ausbeute hergestellte 1-(4-Chlorphenyl)-1-(1,2,4-triazolyl-(1))-2,2-dimethylpentan-1-on schmilzt bei 120 bis 122 °C.

Beispiel 3

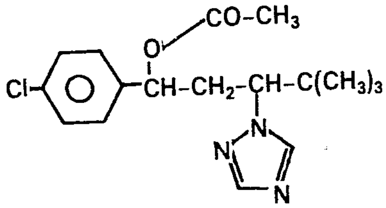

Eine Mischung aus 20,5 g 1-(4-Chlorphenyl)-3-(1,2,4-triazolyl-(1))-4,4-dimethylpentan-1-ol, 1 g Imidazol und 50 g Acetanhydrid werden 3½ Stunden bei 100 °C gerührt und anschliessend in 250 ml Eiswasser eingerührt. Die wässrige Suspension wird dreimal mit je 100 ml Ether ausgeschüttelt. Die vereinigten Etherextrakte werden 15 Minuten mit 200 ml einer 6%igen Natriumhydrogencarbonatlösung gerührt, die organische Phase getrennt, über Natriumsulfat getrocknet und eingeengt. Der feste, farblose Rückstand wird mit Petrolether gerührt und abgesaugt.

Man erhält 18,7 g (85% der Theorie) 1-Acetoxy-1-(4-chlorphenyl)-3-(1,2,4-triazolyl-(1))-4,4-dimethyl-pentan vom Schmelzpunkt 138 bis 140 °C.

Beispiel 4

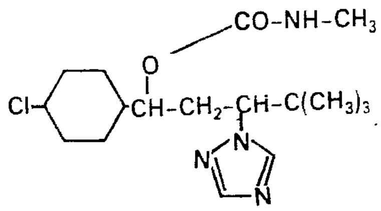

Einer Lösung von 17 g 1-(4-Chlorphenyl)-3-(1,2,4-triazolyl-(1))-4,4-dimethylpentan-1-ol in 50 ml trockenem Tetrahydrofuran werden nacheinander 0,5 ml Triethylamin und 6 g Methylisocyanat bei Raumtemperatur zugetropft.

Nach dreistündigem Rühren wird das Gemisch (4 Stunden bei 50 °C und 0,01 Torr) im Vakuum eingeengt.

Man erhält 20 g (98% der Theorie) 1-(Methylcarbamoyl)-1-(4-chlorphenyl)-3-(1,2,4-triazolyl-(1))-4,4-dimethylpentan als blassgelbes Harz.

In entsprechender Weise können die in der folgenden Tabelle 1 aufgeführten Verbindungen hergestellt werden.

Tabelle

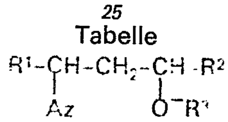

| Beispiel Nr. | $R^1$ | $R^2$ | $R^3$ | Az | Fp/°C | IR (film) [$cm^{-1}$] |
|---|---|---|---|---|---|---|
| 5 | $(CH_3)_3C-$ | $CH_3-$ | $CH_3-$ | 1,2,4-Triazol-1-yl | Öl | 2960, 1499, 1364, 1269, 1137, 1088, 732 |
| 6 | $(CH_3)_3C-$ | $CH_3-$ | $n-C_3H_7$ | 1,2,4-Triazol-1-yl | Öl | 2980, 1505, 1374, 1277, 1147, 1105, 1014, 689 |
| 7 | $(CH_3)_3C-$ | $CH_3-$ | 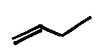 | 1,2,4-Triazol-1-yl | Öl | 2950, 1493, 1362, 1264, 1132, 1058, 1001, 911, 675 |
| 8 | $(CH_3)_3C-$ | $CH_3-$ | $n-C_4H_9$ | 1,2,4-Triazol-1-yl | Öl | 2985, 2895, 1511, 1380, 1282, 1153, 1112, 1022, 693, 675 |
| 9 | $(CH_3)_3C-$ | $CH_3-$ | $n-C_8H_{17}$ | 1,2,4-Triazol-1-yl | Öl | 2950, 2920, 2845, 1496, 1366, 1268, 1135, 1095, 1067, 678 |
| 10 | $(CH_3)_3C-$ | $CH_3-$ | 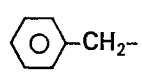 | 1,2,4-Triazol-1-yl | Öl | 2955, 1493, 1449, 1366, 1135, 1090, 732, 695, 678 |
| 11 | $C_6H_5-$ | $CH_3-$ | $C_2H_5-$ | 1,2,4-Triazol-1-yl | Öl | 2960, 1497, 1370, 1270, 1137, 1093, 1075, 1005, 699, 678, 662 |
| 12 | $C_6H_5-$ | $CH_3-$ | 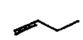 | 1,2,4-Triazol-1-yl | Öl | 2960, 1596, 1268, 1134, 1065, 1003, 920, 727, 696, 677, 660 |
| 13 | $C_6H_5-$ | $CH_3-$ | $n-C_4H_9$ | 1,2,4-Triazol-1-yl | Öl | 2955, 2925, 2865, 1497, 1270, 1136, 1085, 1006, 698, 678, 663 |
| 14 | $C_6H_5-$ | $CH_3-$ | $n-C_8H_{17}$ | 1,2,4-Triazol-1-yl | Öl | 2915, 2845, 1495, 1451, 1373, 1270, 1136, 1094, 1004, 698, 678, 661 |
| 15 | $C_6H_5-$ | $CH_3-$ | 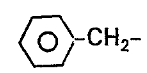 | 1,2,4-Triazol-1-yl | Öl | 2960, 1493, 1449, 1269, 1135, 1092, 1065, 732, 696, 678 |
| 16 | $C_6H_5-$ | $CH_3-$ | 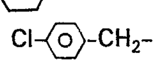 | 1,2,4-Triazol-1-yl | Öl | 2955, 1485, 1267, 1133, 1083, 1010, 803, 696 |
| 17 | $(CH_3)_3C-$ | $C_6H_5$ | $CH_3-$ | 1,2,4-Triazol-1-yl | Harz | 3105, 3020, 2960, 2867, 1500, 1365, 1270, 1198, 1198, 1100, 760, 705, 680 |
| 18 | $(CH_3)_3C-$ | $C_6H_5-$ | 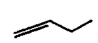 | 1,2,4-Triazol-1-yl | Harz | 3080, 3020, 2950, 2865, 1500, 1368, 1270, 1138, 1005, 764, 703, 681 |

| Beispiel Nr. | $R^1$ | $R^2$ | $R^3$ | Az | Fp/°C | IR (Film) [$cm^{-1}$] |
|---|---|---|---|---|---|---|
| 19 | $(CH_3)_3C-$ | $C_6H_5-$ | 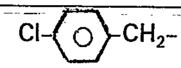 | 1,2,4-Triazol-1-yl | Harz | 3018, 2975, 2860, 1495, 1486, 1270, 1195, 1135, 1084, 1010, 805, 760, 700, 680 |
| 20 | $(CH_3)_2CH-CH_2$ | $C_6H_5-$ | $CH_3-$ | 1,2,4-Triazol-1-yl | Öl | 3020, 2950, 2920, 2860, 1498, 1450, 1270, 1197, 1138, 1100, 1003, 758, 700, 668 |
| 21 | $C_6H_5-$ | $C_6H_5-$ | $CH_3-$ | 1,2,4-Triazol-1-yl | Harz | 3105, 3083, 3060, 3030, 2928, 2820, 1500, 1453, 1273, 1200, 1137, 1100, 760, 700, 576 |
| 22 | $C_6H_5-$ | $C_6H_5-$ | 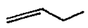 | 1,2,4-Triazol-1-yl | Harz | 3055, 3022, 2920, 2855, 1495, 1450, 1280, 1125, 1004, 698 |
| 23 | $C_6H_5$ | $C_6H_5$ | 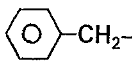 | 1,2,4-Triazol-1-yl | Harz | 3080, 3055, 3023, 2960, 2922, 2884, 1596, 1500, 1450, 1282, 1196, 1085, 1005, 750, 700, 565 |
| 24 | $C_6H_5$ | $C_6H_5$ | 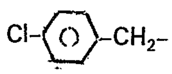 | 1,2,4-Triazol-1-yl | Harz | 3057, 3022, 2920, 2862, 1595, 1478, 1270, 1195, 1135, 1086, 1010, 805, 755, 700, 570 |
| 25 | $(CH_3)_3C-$ | $4-F-C_6H_4-$ | $CH_3-$ | 1,2,4-Triazol-1-yl | Harz | 2955, 1597, 1496, 1267, 1214, 1132, 1090, 832, 676 |
| 26 | $(CH_3)_3C-$ | $4-F-C_6H_4$ | 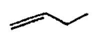 | 1,2,4-Triazol-1-yl | Harz | 2952, 1597, 1495, 1265, 1130, 1073, 1002, 830, 674 |
| 27 | $(CH_3)_3C-$ | $4-F-C_6H_4$ | 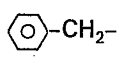 | 1,2,4-Triazol-1-yl | Harz | 2953, 1593, 1490, 1264, 1211, 1130, 1063, 1000, 829, 728, 690, 673 |
| 28 | $(CH_3)_3C-$ | $4-F-C_6H_4$ | 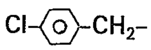 | 1,2,4-Triazol-1-yl | Harz | 2957, 1596, 1493, 1277, 1217, 1132, 1077, 1006, 832, 802, 675 |
| 29 | $(CH_3)_3C-$ | $4-F-C_6H_4$ | 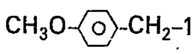 | 1,2,4-Triazol-1-yl | Harz | 2951, 1599, 1495, 1234, 1072, 1027, 830, 675 |
| 30 | 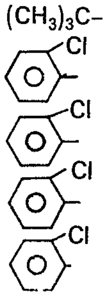 | $CH_3-$ | $CH_2=CH-CH_2-$ | 1,2,4-Triazol-1-yl | Harz | 2965, 1500, 1422, 1272, 1137, 1056, 1036, 1004, 753, 679, 658 |
| 31 | | $CH_3-$ | $n-C_3H_7$ | 1,2,4-Triazol-1-yl | Harz | 2955, 2920, 2855, 1496, 1268, 1133, 1032, 750, 675, 654 |
| 32 | | $CH_3$ | $n-C_4H_9$ | 1,2,4-Triazol-1-yl | Harz | 2945, 2920, 2855, 1593, 1266, 1130, 1085, 1031, 746, 674, 652 |
| 33 | | $CH_3-$ | $n-C_6H_{13}$ | 1,2,4-Triazol-1-yl | Harz | 2950, 2920, 2850, 1496, 1269, 1132, 1089, 748, 676 |
| 34 | 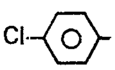 | $CH_3-$ | $C_2H_5-$ | 1,2,4-Triazol-1-yl | Harz | 2965, 1489, 1370, 1271, 1136, 1088, 1011, 845, 677 |
| 35 | 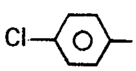 | $CH_3-$ | 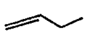 | 1,2,4-Triazol-1-yl | Harz | 2960, 1488, 1371, 1269, 1133, 1086, 1011, 923, 676 |
| 36 | 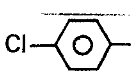 | $CH_3-$ | $n-C_5H_{11}$ | 1,2,4-Triazol-1-yl | Harz | 2950, 2920, 2860, 1588, 1370, 1269, 1135, 1086, 1011, 676 |
| 37 | 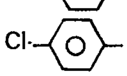 | $CH_3-$ | 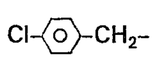 | 1,2,4-Triazol-1-yl | Harz | 2960, 1592, 1484, 1268, 1131, 1083, 1009, 803, 675 |
| 38 | 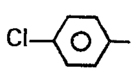 | $CH_3-$ | 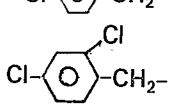 | 1,2,4-Triazol-1-yl | Harz | 1587, 1488, 1469, 1374, 1270, 1135, 1087, 1044, 1011, 813, 676 |
| 39 | 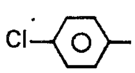 | $(CH_3)_3C-$ | $CH_3-CO-$ | 1,2,4-Triazol-1-yl | 110-112 | |
| 40 | $(CH_3)_3C-$ | 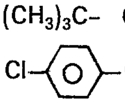 | $CH_3-$ | 1,2,4-Triazol-1-yl | 120-122 | |
| 41 | $(CH_3)_3C-$ | 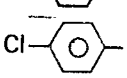 | 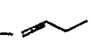 | 1,2,4-Triazol-1-yl | Harz | 3100, 2978, 2860, 1590, 1495, 1484, 1425, 1366, 1268, 1196, 1136, 1085, 1010, 830, 668 |
| 42 | $(CH_3)_2CH\ CH_2-$ | 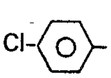 | 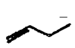 | 1,2,4-Triazol-1-yl | Harz | 3100, 2956, 2865, 1592, 1500, 1486, 1272, 1197, 1138, 1087, 1012, 830, 670, 570 |

| Beispiel Nr. | $R^1$ | $R^2$ | $R^3$ | Az | Fp/°C | IR (Film) [$cm^{-1}$] |
|---|---|---|---|---|---|---|
| 43 | $(C_2H_5)_2CH-$ | 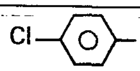 | 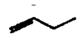 | 1,2,4-Triazol-1-yl | Harz | 2955, 2926, 2870, 1590, 1496, 1433, 1270, 1194, 1136, 1086, 828, 670 |
| 44 | $(CH_3)_3C-$ | 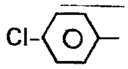 | 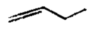 | 1,2,4-Triazol-1-yl | 99–101 | |
| 45 | $(CH_3)_3C-$ | 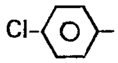 | 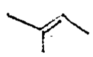 | 1,2,4-Triazol-1-yl | Harz | 2960, 2870, 1592, 1497, 1485, 1368, 1272, 1138, 1085, 1012, 830, 680, 670 |
| 46 | $(CH_3)_3C-$ | 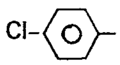 | $HC\equiv C-CH_2-$ | 1,2,4-Triazol-1-yl | Harz | 3118, 2960, 2870, 2115, 1595, 1500, 1488, 1368, 1272, 1200, 1138, 1080, 1015, 830, 660 |
| 47 | $(CH_3)_3C-$ | 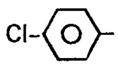 | 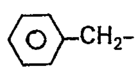 | 1,2,4-Triazol-1-yl | Harz | 3058, 2960, 2868, 1593, 1500, 1486, 1368, 1272, 1198, 1138, 1090, 1070, 1015, 832, 735, 700 |
| 48 | $(CH_3)_3C-$ | 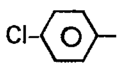 | 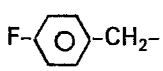 | 1,2,4-Triazol-1-yl | Harz | 2975, 2865, 1598, 1505, 1280, 1220, 1137, 1085, 1010, 825, 680, 670, 500 |
| 49 | $(CH_3)_3C-$ | 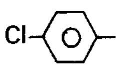 | 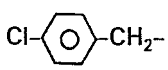 | 1,2,4-Triazol-1-yl | 73–76 | |
| 50 | $(CH_3)_3C-$ | 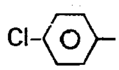 | 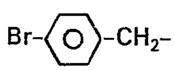 | 1,2,4-Triazol-1-yl | 90–92 | |
| 51 | $(CH_3)_3C-$ | 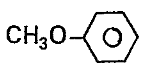 | n-$C_3H_7$- | 1,2,4-Triazol-1-yl | Harz | 2950, 2861, 1605, 1504, 1240, 1166, 1133, 1082, 1029, 828, 675 |
| 52 | $(CH_3)_3C-$ | 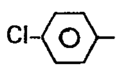 | 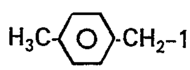 | 1,2,4-Triazol-1-yl | 56–59 | |
| 53 | $(CH_3)_3C-$ | 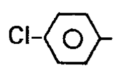 | 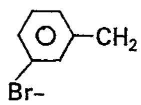 | 1,2,4-Triazol-1-yl | Harz | 2975, 2860, 1590, 1495, 1325, 1268, 1192, 1160, 1120, 1084, 1070, 828, 792, 698 |
| 54 | $(CH_3)_3C-$ | 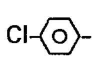 | 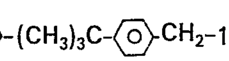 | 1,2,4-Triazol-1-yl | Harz | 2950, 2855, 1590, 1495, 1482, 1470, 1362, 1265, 1192, 1132, 1080, 830, 677, 665, 572 |
| 55 | $(CH_3)_3C-$ | 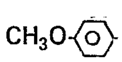 | $CH_3-CO-$ | 1,2,4-Triazol-1-yl | Harz | 2952, 1730, 1606, 1509, 1366, 1235, 1171, 1027, 829, 678 |
| 56 | $(CH_3)_3C-$ | 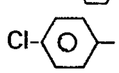 | $CH_3-CH_2-CO-$ | 1,2,4-Triazol-1-yl | 78–80 | |
| 57 | $(CH_3)_3C-$ | 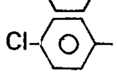 | $CH_3-CO-$<br>$CH_2-CO-$ | 1,2,4-Triazol-1-yl | Harz | 3110, 2974, 1736, 1710, 1496, 1487, 1360, 1310, 1135, 1085, 1009, 825, 678 |
| 58 | $(CH_3)_3C-$ | 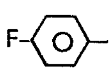 | $CH_3-CO-$ | 1,2,4-Triazol-1-yl | 88–91 | |
| 59 | $(CH_3)_3C-$ | 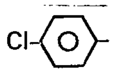 | 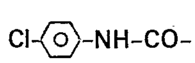 | 1,2,4-Triazol-1-yl | Harz | 3115, 2960, 1725, 1596, 1490, 1305, 1220, 1090, 1011, 826, 680, 666, 508 |
| 60 | $(CH_3)_3C-$ | 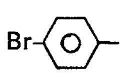 | $CH_3-$ | 1,2,4-Triazol-1-yl | 118–120 | |
| 61 | $(CH_3)_3C-$ | 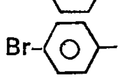 | 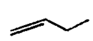 | 1,2,4-Triazol-1-yl | Öl | 3100, 2960, 2870, 1588, 1500, 1482, 1366, 1270, 1136, 1075, 1010, 825, 689, 665, 570 |
| 62 | $(CH_3)_3C-$ | 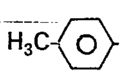 | $CH_3-$ | 1,2,4-Triazol-1-yl | 90–91 | |
| 63 | $(CH_3)_3C-$ | 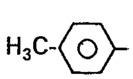 | 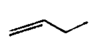 | 1,2,4-Triazol-1-yl | Harz | 2950, 2860, 1510, 1500, 1270, 1198, 1137, 1080, 1006, 923, 820, 682, 665, 582 |

| Beispiel Nr. | R[1] | R[2] | R[3] | Az | Fp/°C | IR (Film) [cm-1] |
|---|---|---|---|---|---|---|
| 64 | $(CH_3)_3C-$ | 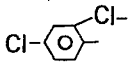 | $CH_3-$ | 1,2,4-Triazol-1-yl | 84–86 | |
| 65 | $(CH_3)_3C-$ | 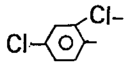 | 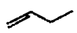 | 1,2,4-Triazol-1-yl | Öl | 2960, 2865, 1586, 1500, 1466, 1368, 1270, 1138, 1090, 1042, 824, 680, 590 |
| 66 | $(CH_3)_3C-$ | 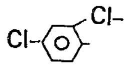 | $HC{=}CH_2-$ | 1,2,4-Triazol-1-yl | 148–150 | |
| 67 | $(CH_3)_3C-$ | 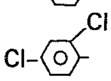 | 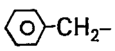 | 1,2,4-Triazol-1-yl | Harz | 3025, 2955, 2865, 1584, 1498, 1466, 1368, 1270, 1136, 1085, 823, 740, 699 |
| 68 | 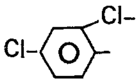 | $(CH_3)_3C-$ | $CH_3-CO-$ | Imidazol-1-yl | Harz | 3100, 2966, 1728, 1582, 1460, 1365, 1230, 1103, 1040, 1018, 815, 658 |
| 69 | 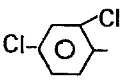 | $(CH_3)_3C-$ | 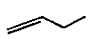 | 1,2,4-Triazol-1-yl | Harz | 3080, 2950, 2860, 1586, 1558, 1498, 1470, 1271, 1192, 1135, 923, 822, 680 |
| 70 | 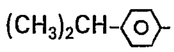 | $(CH_3)_3C-$ | $CH_3-NH-CO-$ | 1,2,4-Triazol-1-yl | Harz | 2955, 2870, 1705, 1500, 1418, 1362, 1260,1135, 1000, 956, 770, 678, 658 |
| 71 | 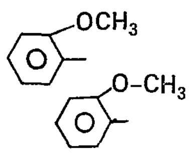 | $(CH_3)_3C-$ | 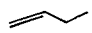 | 1,2,4-Triazol-1-yl | Harz | 2950, 1490, 1243, 1135, 1072, 752, 662 |
| 72 | | $(CH_3)_3C-$ | $n-C_4H_9$ | 1,2,4-Triazol-1-yl | Harz | 2940, 1597, 1488, 1460, 1267, 1240, 1132, 1090, 1022, 1004, 752, 661 |
| 73 | 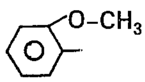 | $(CH_3)_3C-$ | $CH_3-CO-$ | 1,2,4-Triazol-1-yl | Harz | 2965, 1736, 1604, 1496, 1467, 1374, 1248, 1140, 1025, 759, 685, 667 |
| 74 | 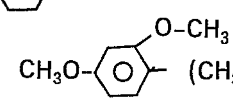 | $(CH_3)_3-$ | $Cl-CH_2-CO-$ | 1,2,4-Triazol-1-yl | Harz | 2955, 1750, 1491, 1286, 1248, 1165, 980, 754 |
| 75 | 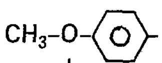 | $(CH_3)_3C-$ | $CH_3-CO-$ | 1,2,4-Triazol-1-yl | 103–105 | |
| 76 | 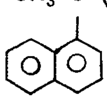 | $CH_3-$ | $CH_3-$ | 1,2,4-Triazol-1-yl | Harz | 2960, 1496, 1440, 1270, 1201, 1134, 1055, 742, 696, 678 |
| 77 | 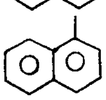 | $CH_3-$ | $n-C_4H_9$ | 1,2,4-Triazol-1-yl | Harz | 2945, 2915, 2855, 1595, 1267, 1132, 1089, 773, 676 |
| 78 | 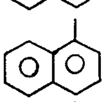 | $CH_3-$ | $-CH_2-C_6H_5$ | 1,2,4-Triazol-1-yl | Harz | 2965, 2925, 1495, 1452, 1272, 1135, 1060, 777, 736, 699 |
| 79 | 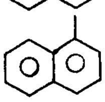 | $(CH_3)_3C-$ | 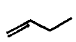 | 1,2,4-Triazol-1-yl | Harz | 2950, 2865, 1498, 1363, 1272, 1135, 1082, 1050, 1005, 993, 977, 679 |
| 80 | $(CH_3)_3C-$ | 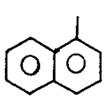 | $CH_3-$ | 1,2,4-Triazol-1-yl | 124–125 | |
| 81 | $(CH_3)_3C-$ | 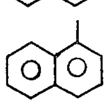 | 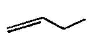 | 1,2,4-Triazol-1-yl | Harz | 3050, 2955, 2860, 1590, 1498, 1365, 1270, 1135, 1080, 800, 775, 680 |
| 82 | $(CH_3)_3C-$ | 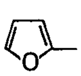 | $CH_3-$ | 1,2,4-Triazol-1-yl | 53–55 | |
| 83 | $(CH_3)_3C-$ | 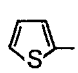 | $CH_3$ | 1,2,4-Triazol-1-yl | Harz | 3090, 2950, 2810, 1493, 1362, 1266, 1132, 1080, 1003, 700, 675, 660 |

| Beispiel Nr. | $R^1$ | $R^2$ | $R^3$ | Az | Fp/°C | IR (Film) [$cm^{-1}$] |
|---|---|---|---|---|---|---|
| 84 | $(CH_3)_3C$- | 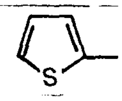 | 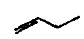 | 1,2,4-Triazol-1-yl | Harz | 3100, 2960, 2865, 1498, 1366, 1270, 1136, 1075, 925, 700, 680, 665 |
| 85 | $(CH_3)_3C$- | 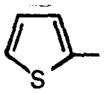 | 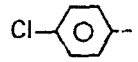 | 1,2,4-Triazol-1-yl | Harz | 3100, 2960, 2870, 1595, 1500, 1490, 1366, 1270, 1136, 1080, 1012, 700 |
| 86 | 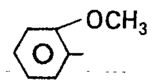 | $CH_3$- | $C_2H_5$ | 1,2,4-Triazol-1-yl | Harz | 2965, 1491, 1460, 1245, 1150, 1090, 1025, 752 |
| 87 | 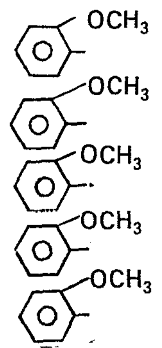 | $CH_3$- | 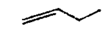 | 1,2,4-Triazol-1-yl | Harz | 2952, 1596, 1488, 1458, 1267, 1241, 1135, 1022, 752 |
| 88 | | $CH_3$- | n-$C_4H_9$ | 1,2,4-Triazol-1-yl | Harz | 2952, 2925, 1492, 1461, 1270, 1244, 1187, 1088, 1025, 751 |
| 89 | | $CH_3$- | n-$C_6H_{13}$ | 1,2,4-Triazol-1-yl | Harz | 2920, 1490, 1460, 1269, 1242, 1134, 1088, 1023, 751, 676, 659 |
| 90 | | $CH_3$- | n-$C_8H_{17}$ | 1,2,4-Triazol-1-yl | Harz | 2918, 1490, 1460, 1269, 1242, 1134, 1090, 1025, 751 |
| 91 | | $CH_3$ | 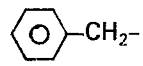 | 1,2,4-Triazol-1-yl | Harz | 2910, 1486, 1457, 1370, 1239, 1131, 1021, 746, 691 |
| 92 | 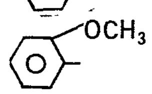 | $CH_3$- | 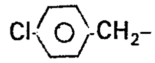 | 1,2,4-Triazol-1-yl | Harz | 2952, 2920, 2850, 1596, 1488, 1404, 1240, 1181, 1011, 804, 751 |
| 93 | 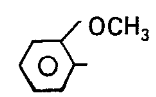 | $CH_3$- | $(CH_3)_3C$-CO- | 1,2,4-Triazol-1-yl | Harz | 2960, 1721, 1490, 1460, 1278, 1242, 1160, 1135, 1024, 752 |

In den nachfolgenden Beispielen wird die Wirkung der erfindungsgemäss verwendbaren Stoffe als Pflanzenwachstumsregulatoren dargestellt, ohne die Möglichkeit weiterer Anwendungen als Wachstumsregulatoren auszuschliessen.

Zur Bestimmung der wachstumsregulierenden Eigenschaft der Prüfsubstanzen wurden Testpflanzen auf ausreichend mit Nährstoffen versorgtem Torfkultursubstrat in Kunststoffgefässen von ca. 12,5 cm Durchmesser unter Gewächshausbedingungen herangezogen. Im Vorauflaufverfahren wurden die zu testenden Substanzen in verschiedenen Konzentrationen als wässrige Aufbereitungen am Tage der Aussaat auf das Saatbett gegossen. Bei der Blattbehandlung, die bei einer Wuchshöhe von ca. 10 cm erfolgte, wurden die Pflanzen mit wässrigen Aufbereitungen der Prüfsubstanzen in unterschiedlichen Konzentrationen besprüht. Die beobachtete wachstumsregulierende Wirkung der geprüften Substanzen wurde zu Versuchsende durch Wuchshöhenmessung belegt. Die so gewonnenen Messwerte wurden zur Wuchshöhe der unbehandelten Pflanzen in Relation gesetzt. Als Vergleichssubstanzen dienten die bekannten Verbindungen CCC und A.

Vergleichssubstanzen:

CCC 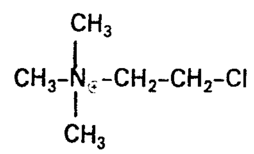 $Cl^-$

A 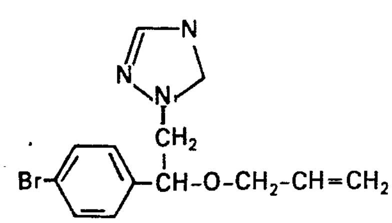 $HNO_3$

1. Einfluss auf das Längenwachstum von monokotylen Pflanzen

1.1 Sommerweizen, Sorte «Kolibri», Vorauflaufverfahren
Versuchsdauer: 14 Tage

| Wirkstoff Nr. | Konzentration mg Wirkstoff/Gef. | Wuchshöhen cm | % |
|---|---|---|---|
| unbehandelt (Kontrolle) | - | 28,8 | 100 |
| CCC | 3 | 20,5 | 71,2 |
| | 12 | 16,0 | 55,6 |
| 17 | 3 | 16,5 | 57,3 |
| | 12 | 7,0 | 24,3 |

| Wirkstoff Nr. | Konzentration mg Wirkstoff/Gef. | Wuchshöhen cm | % |
|---|---|---|---|
| 18 | 3 | 20,5 | 71,2 |
| | 12 | 14,0 | 48,6 |

1.2 Sommerweizen, Sorte «Kolibri»
Vorauflaufverfahren
Versuchsdauer: 14 Tage

| Wirkstoff Nr. | Konzentration mg Wirkstoff/Gef. | Wuchshöhen cm | % |
|---|---|---|---|
| unbehandelt (Kontrolle) | - | 30,4 | 100 |
| CCC | 3 | 20,0 | 65,8 |
| | 12 | 19,0 | 62,5 |
| 3 | 3 | 20,0 | 65,8 |
| | 12 | 17,0 | 55,9 |
| 58 | 3 | 17,5 | 57,6 |
| | 12 | 15,0 | 49,3 |

1.3 Sommerweizen, Sorte «Kolibri»,
Vorauflaufverfahren
Versuchsdauer: 15 Tage

| Wirkstoff Nr. | Konzentration mg Wirkstoff/Gef. | Wuchshöhen cm | % |
|---|---|---|---|
| unbehandelt (Kontrolle) | - | 31,3 | 100 |
| CCC | 3 | 22,0 | 70,3 |
| | 12 | 20,5 | 65,5 |
| 34 | 3 | 18,0 | 57,5 |
| | 12 | 6,0 | 19,2 |

1.4 Sommerweizen, Sorte «Kolibri»,
Vorauflaufverfahren
Versuchsdauer: 12 Tage

| Wirkstoff Nr. | Konzentration mg Wirkstoff/Gef. | Wuchshöhen cm | % |
|---|---|---|---|
| unbehandelt (Kontrolle) | - | 27,8 | 100 |
| CCC | 3 | 22,5 | 80,9 |
| | 12 | 19,0 | 68,4 |
| A | 3 | 26,0 | 93,5 |
| | 12 | 19,0 | 68,4 |
| 56 | 3 | 21,5 | 77,3 |
| | 12 | 15,0 | 54,0 |

1.5 Sommerweizen, Sorte «Kolibri»,
Vorauflaufverfahren
Versuchsdauer: 14 Tage

| Wirkstoff Nr. | Konzentration mg Wirkstoff/Gef. | Wuchshöhen cm | % |
|---|---|---|---|
| unbehandelt (Kontrolle) | - | 30,4 | 100 |
| CCC | 3 | 20,0 | 65,8 |
| | 12 | 19,0 | 62,5 |
| 57 | 3 | 20,0 | 65,8 |
| | 12 | 16,0 | 52,6 |

1.6 Sommergerste, Sorte «Union»,
Vorauflaufverfahren
Versuchsdauer: 14 Tage

| Wirkstoff Nr. | Konzentration mg Wirkstoff/Gef. | Wuchshöhen cm | % |
|---|---|---|---|
| unbehandelt (Kontrolle) | - | 28,3 | 100 |
| CCC | 3 | 24,0 | 84,8 |
| | 12 | 22,0 | 77,7 |
| 17 | 3 | 13,0 | 45,9 |
| | 12 | 5,0 | 17,7 |
| 18 | 3 | 25,0 | 88,3 |
| | 12 | 14,0 | 49,5 |

1.7 Sommergerste, Sorte «Union»,
Vorauflaufverfahren
Versuchsdauer: 14 Tage

| Wirkstoff Nr. | Konzentration mg Wirkstoff/Gef. | Wuchshöhen cm | % |
|---|---|---|---|
| unbehandelt (Kontrolle) | - | 25,7 | 100 |
| CCC | 3 | 21,0 | 81,7 |
| | 12 | 20,0 | 77,8 |
| 25 | 3 | 22,0 | 85,3 |
| | 12 | 17,0 | 65,9 |

1.8 Hafer, Sorte «Flämingskrone»,
Vorauflaufverfahren
Versuchsdauer: 16 Tage

| Wirkstoff Nr. | Konzentration mg Wirkstoff/Gef. | Wuchshöhen cm | % |
|---|---|---|---|
| unbehandelt (Kontrolle) | - | 25,8 | 100 |
| CCC | 3 | 24,0 | 93,0 |
| | 12 | 22,5 | 87,2 |
| 25 | 3 | 22,0 | 85,3 |
| | 12 | 17,0 | 65,9 |
| 40 | 3 | 21,0 | 81,4 |
| | 12 | 19,0 | 73,6 |

2. Einfluss auf das Längenwachstum von Dikotylen

2.1 Sommerraps, Sorte «Petronova», Vorauflaufverfahren
Versuchsdauer: 16 Tage

| Wirkstoff Nr. | Konzentration mg Wirkstoff/Gef. | Wuchshöhen cm | % |
|---|---|---|---|
| unbehandelt (Kontrolle) | - | 20,0 | 100 |
| CCC | 3 | 18,0 | 90,0 |
| | 12 | 17,5 | 87,5 |
| 3 | 3 | 15,5 | 77,5 |
| | 12 | 14,0 | 70,0 |
| 58 | 3 | 17,5 | 87,5 |
| | 12 | 15,0 | 75,0 |

2.2 Sommerraps, Sorte «Cosa», Vorauflaufverfahren
Versuchsdauer: 16 Tage

| Wirkstoff Nr. | Konzentration mg Wirkstoff/Gef. | Wuchshöhen cm | % |
|---|---|---|---|
| unbehandelt (Kontrolle) | - | 17,3 | 100 |
| CCC | 3 | 16,5 | 95,4 |
| | 12 | 16,5 | 95,4 |
| 30 | 3 | 15,5 | 89,6 |
| | 12 | 14,5 | 83,8 |
| 31 | 3 | 15,5 | 89,6 |
| | 12 | 13,0 | 75,1 |
| 32 | 3 | 15,5 | 89,6 |
| | 12 | 13,5 | 78,0 |

2.3 Sommerraps, Sorte «Cosa», Vorauflaufverfahren
Versuchsdauer: 19 Tage

| Wirkstoff Nr. | Konzentration mg Wirkstoff/Gef. | Wuchshöhen cm | % |
|---|---|---|---|
| unbehandelt (Kontrolle) | - | 18,0 | 100 |
| CCC | 3 | 16,5 | 91,7 |
| | 12 | 16,5 | 91,7 |
| 34 | 3 | 16,5 | 91,7 |
| | 12 | 15,3 | 85,0 |

2.4 Sommerraps, Sorte «Petronova», Vorauflaufverfahren
Versuchsdauer: 16 Tage

| Wirkstoff Nr. | Konzentration mg Wirkstoff/Gef. | Wuchshöhen cm | % |
|---|---|---|---|
| unbehandelt (Kontrolle) | - | 14,8 | 100 |
| CCC | 3 | 14,0 | 94,6 |
| | 12 | 13,0 | 87,8 |
| 56 | 3 | 13,5 | 91,2 |
| | 12 | 10,5 | 71,0 |

2.5 Sommerraps, Sorte «Petronova», Vorauflaufverfahren
Versuchsdauer: 16 Tage

| Wirkstoff Nr. | Konzentration mg Wirkstoff/Gef. | Wuchshöhen cm | % |
|---|---|---|---|
| unbehandelt | - | 20,0 | 100 |
| CCC | 3 | 18,0 | 90,0 |
| | 12 | 17,5 | 87,5 |
| 57 | 3 | 16,5 | 82,5 |
| | 12 | 14,0 | 70,0 |

2.6 Sommerraps, Sorte «Cosa», Blattbehandlung
Versuchsdauer: 19 Tage

| Wirkstoff Nr. | Konzentration mg Wirkstoff/Gef. | Wuchshöhen cm | % |
|---|---|---|---|
| unbehandelt (Kontrolle) | - | 19,4 | 100 |
| CCC | 1,5 | 17,0 | 87,6 |
| | 6 | 16,0 | 82,5 |
| 36 | 1,5 | 16,5 | 85,1 |
| | 6 | 14,0 | 72,2 |
| 37 | 1,5 | 16,5 | 85,1 |
| | 6 | 15,5 | 79,9 |

2.7 Sommerraps, Sorte «Petronova», Blattbehandlung
Versuchsdauer: 19 Tage

| Wirkstoff Nr. | Konzentration mg Wirkstoff/Gef. | Wuchshöhen cm | % |
|---|---|---|---|
| unbehandelt (Kontrolle) | - | 17,1 | 100 |
| CCC | 1,5 | 16,0 | 93,6 |
| | 6 | 15,5 | 90,6 |
| A | 1,5 | 17,0 | 99,4 |
| | 6 | 15,5 | 90,6 |
| 69 | 1,5 | 13,8 | 80,7 |
| | 6 | 12,3 | 71,9 |

Beispiel 94

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel 95

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100000 Gewichtsteilen Wasser erhält man eine wässrige Dispersion, die 0,02 Gewichtsprozent des Wirkstoffs enthält.

Beispiel 96

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingiessen und feines Verteilen der Lösung in 100000 Gewichtsteilen Wasser erhält man eine wässrige Dispersion, die 0,02 Gewichtsprozent des Wirkstoffs enthält.

Beispiel 97

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingiessen und feines Verteilen der Lösung in 100000 Gewichtsteilen Wasser erhält man eine wässrige Dispersion, die 0,02 Gewichtsprozent des Wirkstoffs enthält.

Beispiel 98

20 Gewichtsteile des Wirkstoffs 2 werden mit 3 Gewichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gewichtsprozent des Wirkstoffs enthält.

Beispiel 99

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 100

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 101

40 Gewichtsteile des Wirkstoffs 1 werden mit 10 Teilen Natriumsalz eines Phenolsulfonsäureharnstoff-formaldehyd-Kondensats, 2 Teilen Kieselgel und 48 Teilen Wasser innig vermischt. Man erhält eine stabile wässrige Dispersion. Durch Verdünnen mit 100000 Gewichsteilen Wasser erhält man eine wässrige Dispersion, die 0,04 Gewichtsprozent Wirkstoff enthält.

Beispiel 102

20 Teile des Wirkstoffs 2 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche für die Vertragsstaaten: DE, FR, GB, IT, BE, CH, NL, SE, LU**

1. γ-Azolylverbindungen der allgemeinen Formel

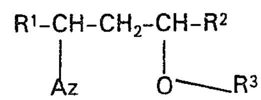

I

in der $R^1$ und $R^2$ verschieden oder gleich sind und Alkyl mit 1 bis 8 C-Atomen, Furanyl, Thienyl, Naphthyl oder Phenyl bedeuten, wobei der Phenylrest durch Fluor, Chlor, Brom, Nitro, Trifluormethyl, ferner durch Alkyl, Alkoxy oder Alkenyl mit 1 bis 4 C-Atomen substituiert sein kann und $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl, ferner durch Alkyl oder Alkoxy mit 1 bis 4 C-Atomen substituiert sein kann oder $R^3$ einen $-CO-R^4$ Rest bedeutet, wobei $R^4$ einen gegebenenfalls durch Halogen, Alkoxy, Oxo (= O) oder Carboxyalkyl substituierten Alkylrest mit 1 bis 5 C-Atomen oder einen Phenylrest bedeutet und $R^3$ ferner einen $-CO-NH-R^5$ Rest bedeutet, wobei $R^5$ einen Alkylrest mit 1 bis 4 C-Atomen oder einen Chlorphenylrest bedeutet und

Az einen über ein Ringstickstoffatom gebundenen Imidazolyl-, 1,2,4-Triazolyl- oder 1,2,3-Triazolylrest bedeutet.

2. Wachstumsregulierendes Mittel, enthaltend einen festen oder flüssigen Trägerstoff und eine γ-Azolylverbindung wie in Anspruch 1 definiert.

3. Verfahren zur Herstellung eines wachstumsregulierenden Mittels, dadurch gekennzeichnet, dass man einen festen oder flüssigen Trägerstoff vermischt mit einer γ-Azolylverbindung gemäss Anspruch 1.

4. Verfahren zur Regulierung des Pflanzenwachstums, dadurch gekennzeichnet, dass man die Pflanzen oder ihre Samen oder den Boden behandelt mit einer γ-Azolylverbindung gemäss Anspruch 1.

5. γ-Azolylverbindung gemäss Anspruch 1, ausgewählt aus der Gruppe bestehend aus
1-Acetoxy-1-(4-chlorphenyl)-3-(1,2,4-triazolyl-(1))-4,4-dimethylpentan,
1-Methoxy-1-phenyl-3-(1,2,4-triazolyl-(1))-4,4-dimethylpentan,
1-Allyloxy-1-phenyl-3-(1,2,4-triazolyl-(1))-4,4-dimethylpentan,
1-Methoxy-1-(4-fluorphenyl)-3-(1,2,4-triazolyl-(1))-4,4-dimethylpentan,
1-(2-Chlorphenyl)-1-(1,2,4-triazolyl-(1))-3-allyloxy-butan,
1-(2-Chlorphenyl)-1-(1,2,4-triazolyl-(1))-3-(1-propoxy)-butan,
1-(2-Chlorphenyl)-1-(1,2,4-triazolyl-(1))-3-(1-butoxy)-butan,
1-(4-Chlorphenyl)-1-(1,2,4-triazolyl-(1))-3-ethoxy-butan,
1-(4-Chlorphenyl)-1-(1,2,4-triazolyl-(1))-3-(1-pentyloxy)-butan,
1-(4-Chlorphenyl)-1-(1,2,4-triazolyl-(1))-3-(4-chlorbenzyl)-butan,
1-Methoxy-1-(4-chlorphenyl)-3-(1,2,4-triazolyl-(1))-4,4-dimethylpentan,
1-Acetoxy-1-(4-Chlorphenyl)-3-(1,2,4-triazolyl-(1))-4,4-dimethylpentan,
1-Propionyloxy-1-(4-chlorphenyl)-3-(1,2,4-triazolyl-(1))-4,4-dimethylpentan,
1-Acetylacetoxy-1-(4-chlorphenyl)-3-(1,2,4-triazolyl-(1))-4,4-dimethylpentan,
1-Acetoxy-1-(4-fluorphenyl)-3-(1,2,4-triazolyl-(1))-4,4-dimethylpentan,
1-(2,4-Dichlorphenyl)-1-(1,2,4-triazolyl-(1))-3-allyloxy-4,4-dimethylpentan.

6. Wachstumsregulierendes Mittel, enthaltend eine γ-Triazolylverbindung gemäss Anspruch 5.

**Patentansprüche für den Vertragsstaat: Österreich**

1. Wachstumsregulierende Mittel, enthaltend eine γ-Azolylverbindung der allgemeinen Formel

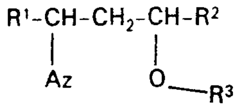

in der $R^1$ und $R^2$ verschieden oder gleich sind und Alkyl mit 1 bis 8 C-Atomen, Furanyl, Thienyl, Naphthyl oder Phenyl bedeuten, wobei der Phenylrest durch Fluor, Chlor, Brom, Nitro, Trifluormethyl, ferner durch Alkyl, Alkoxy oder Alkenyl mit 1 bis 4 C-Atomen substituiert sein kann und $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl, ferner durch Alkyl oder Alkoxy mit 1 bis 4 C-Atomen substituiert sein kann oder $R^3$ einen $-CO-R^4$ Rest bedeutet, wobei $R^4$ einen gegebenenfalls durch Halogen, Alkoxy, Oxo (= O) oder Carboxyalkyl substituierten Alkylrest mit 1 bis 5 C-Atomen oder einen Phenylrest bedeutet und $R^3$ ferner einen $-CO-NH-R^5$ Rest bedeutet, wobei $R^5$ einen Alkylrest mit 1 bis 4 C-Atomen oder einen Chlorphenylrest bedeutet und
Az einen über ein Ringstickstoffatom gebundenen Imidazolyl-, 1,2,4-Triazolyl- oder 1,2,3-Triazolylrest bedeutet.

2. Wachstumsregulierendes Mittel, enthaltend einen festen oder flüssigen Trägerstoff und eine γ-Azolylverbindung wie in Anspruch 1 definiert.

3. Verfahren zur Herstellung eines wachstumsregulierenden Mittels, dadurch gekennzeichnet, dass man einen festen oder flüssigen Trägerstoff vermischt mit einer γ-Azolylverbindung wie in Anspruch 1 definiert.

4. Verfahren zur Regulierung des Pflanzenwachstums, dadurch gekennzeichnet, dass man die Pflanzen oder ihre Samen oder den Boden behandelt mit einer γ-Azolylverbindung wie in Anspruch 1 definiert.

5. Wachstumsregulierendes Mittel gemäss Anspruch 1, enthaltend eine γ-Triazolylverbindung, ausgewählt aus der Gruppe bestehend aus
1-Acetoxy-1-(4-chlorphenyl)-3-(1,2,4-triazolyl-(1))-4,4-dimethylpentan,
1-Methoxy-1-phenyl-3-(1,2,4-triazolyl-(1))-4,4-dimethylpentan,
1-Allyloxy-1-phenyl-3-(1,2,4-triazolyl-(1))-4,4-dimethylpentan,
1-Methoxy-1-(4-fluorphenyl)-3-(1,2,4-triazolyl-(1))-4,4-dimethylpentan,
1-(2-Chlorphenyl)-1-(1,2,4-triazolyl-(1))-3-allyloxy-butan,
1-(2-Chlorphenyl)-1-(1,2,4-triazolyl-(1))-3-(1-propoxy)-butan,
1-(2-Chlorphenyl)-1-(1,2,4-triazolyl-(1))-3-(1-butoxy)-butan,
1-(4-Chlorphenyl)-1-(1,2,4-triazolyl-(1))-3-ethoxy-butan,
1-(4-Chlorphenyl)-1-(1,2,4-triazolyl-(1))-3-(1-pentyloxy)-butan,
1-(4-Chlorphenyl)-1-(1,2,4-triazolyl-(1))-3-(4-chlorbenzyl)-butan,
1-Methoxy-1-(4-chlorphenyl)-3-(1,2,4-triazolyl-(1))-4,4-dimethylpentan,
1-Acetoxy-1-(4-Chlorphenyl)-3-(1,2,4-triazolyl-(1))-4,4-dimethylpentan,
1-Propionyloxy-1-(4-chlorphenyl)-3-(1,2,4-triazolyl-(1))-4,4-dimethylpentan,
1-Acetylacetoxy-1-(4-chlorphenyl)-3-(1,2,4-triazolyl-(1))-4,4-dimethylpentan,
1-Acetoxy-1-(4-fluorphenyl)-3-(1,2,4-triazolyl-(1))-4,4-dimethylpentan,
1-(2,4-Dichlorphenyl)-1-(1,2,4-triazolyl-(1))-3-allyloxy-4,4-dimethylpentan.

**Claims for the Contracting states: BE, CH, DE, FR, GB, IT, LU, NL, SE**

1. A γ-azolyl compound of the general formula

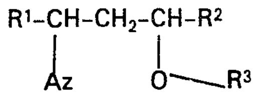

I,

where $R^1$ and $R^2$ are identical or different and each denotes alkyl of 1 to 8 carbon atoms, furanyl, thienyl, naphthyl, or phenyl, phenyl being unsubstituted or substituted by fluoro, chloro, bromo, nitro, trifluoromethyl, alkyl of 1 to 4 carbon atoms, alkoxy of 1 to 4 carbon atoms or alkenyl of 1 to 4 carbon atoms, $R^3$ denotes alkyl of 1 to 8 carbon atoms, unsubstituted or chloro-substituted alkenyl of 2 to 5 carbon atoms, alkynyl of 3 to 4 carbon atoms, benzyl which is unsubstituted or substituted by fluoro, chloro, bromo, nitro, trifluoromethyl, or by alkyl or alkoxy of 1 to 4 carbon atoms, $R^3$ further denotes $-CO-R^4$, $R^4$ denoting alkyl of 1 to 5 carbon atoms which is unsubstituted or substituted by halogen, alkoxy, oxo (= O) or carboxyalkyl, or $R^4$ denoting phenyl, and $R^3$ further denotes $-CO-NH-R^5$, $R^5$ denoting alkyl of 1 to 4 carbon atoms or chlorophenyl, and Az denotes imidazolyl, 1,2,4-triazolyl or 1,2,3-triazolyl attached via a ring nitrogen atom.

2. A plant growth regulator containing a γ-azolyl compound as claimed in claim 1.

3. A process for the manufacture of a plant growth regulator, characterized in that a solid or liquid carrier is mixed with a γ-azolyl compound as claimed in claim 1.

4. A process for regulating plant growth, characterized in that the plants, their seed, or the soil are treated with a γ-azolyl compound as claimed in claim 1.

5. A γ-azolyl compound as claimed in claim 1, selected from the group consisting of
1-acetoxy-1-(4-chlorophenyl)-3-(1,2,4-triazolyl-(1))-4,4-dimethylpentane,
1-methoxy-1-phenyl-3-(1,2,4-triazolyl-(1))-4,4-dimethylpentane,
1-allyloxy-1-phenyl-3-(1,2,4-triazolyl-(1))-4,4-diemthylpentane,
1-methoxy-1-(4-fluorophenyl)-3-(1,2,4-triazolyl-(1))-4,4-dimethylpentane,
1-(2-chlorophenyl)-1-(1,2,4-triazolyl-(1))-3-allyloxybutane,
1-(2-chlorophenyl)-1-(1,2,4-triazolyl-(1))-3-(1-propoxy)-butane,
1-(2-chlorophenyl)-1-(1,2,4-triazolyl-(1))-3-(1-butoxy)-butane,
1-(4-chlorophenyl)-1-(1,2,4-triazolyl-(1))-3-ethoxybutane,
1-(4-chlorophenyl)-1-(1,2,4-triazolyl-(1))-3-(1-pentyloxy)-butane,
1-(4-chlorophenyl)-1-(1,2,4-triazolyl-(1))-3-(4-chlorobenzyl)-butane,
1-methoxy-1-(4-chlorophenyl)-3-(1,2,4-triazolyl-(1))-4,4-dimethylpentane,
1-acetoxy-1-(4-chlorophenyl)-3-(1,2,4-triazolyl-(1))-4,4-dimethylpentane,
1-propionyloxy-1-(4-chlorophenyl)-3-(1,2,4-triazolyl-(1))-4,4-dimethylpentane,
1-acetylacetoxy-1-(4-chlorophenyl)-4-(1,2,4-triazolyl-(1))-4,4-dimethylpentane,
1-acetoxy-1-(4-fluorophenyl)-3-(1,2,4-triazolyl-(1))-4,4-dimethylpentane, and
1-(2,4-dichlorophenyl)-1-(1,2,4-triazolyl-(1))-3-allyloxy-4,4-dimethylpentane.

6. A plant growth regulator containing a γ-triazolyl compound as claimed in claim 5.

**Claims for the Contracting state: AT**

1. A plant growth regulator containing a γ-azolyl compound of the general formula

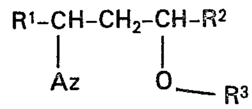

where $R^1$ and $R^2$ are identical or different and each denotes alkyl of 1 to 8 carbon atoms, furanyl, thienyl, naphthyl, or phenyl, phenyl being unsubstituted or substituted by fluoro, chloro, bromo, nitro, trifluoromethyl, alkyl of 1 to 4 carbon atoms, alkoxy of 1 to 4 carbon atoms or alkenyl of 1 to 4 carbon atoms, $R^3$ denotes alkyl of 1 to 8 carbon atoms, unsubstituted or chloro-substituted alkenyl of 2 to 5 carbon atoms, alkynyl of 3 to 4 carbon atoms, benzyl which is unsubstituted or substituted by fluoro, chloro, bromo, nitro, trifluoromethyl, or by alkyl or alkoxy of 1 to 4 carbon atoms, $R^3$ further denotes $-CO-R^4$, $R^4$ denoting alkyl of 1 to 5 carbon atoms which is unsubsituted or substituted by halogen, alkoxy, oxo (= O) or carboxyalkyl, or $R^4$ denoting phenyl, and $R^3$ further denotes $-CO-NH-R^5$, $R^5$ denoting alkyl of 1 to 4 carbon atoms or chlorophenyl, and Az denotes imidazolyl, 1,2,4-triazolyl or 1,2,3-triazolyl attached via a ring nitrogen atom.

2. A plant growth regulator containing a solid or liquid carrier and a γ-azolyl compound as defined in claim 1.

3. A process for the manufacture of a plant growth regulator, characterized in that a solid or liquid carrier is mixed with an γ-azolyl compound as defined in claim 1.

4. A process for regulating plant growth, characterized in that the plants, their seed, or the soil are treated with a γ-azolyl compound as defined in claim 1.

5. A plant growth regulator as claimed in claim 1, containing a γ-azolyl compound selected from the group consisting of
1-acetoxy-1-(4-chlorophenyl)-3-(1,2,4-triazolyl-(1))-4,4-dimethylpentane,
1-methoxy-1-phenyl-3-(1,2,4-triazolyl-(1))-4,4-dimethylpentane,
1-allyloxy-1-phenyl-3-(1,2,4-triazolyl-(1))-4,4-dimethylpentane,
1-methoxy-1-(4-fluorophenyl)-3-(1,2,4-triazolyl-(1))-4,4-dimethylpentane,
1-(2-chlorophenyl)-1-(1,2,4-triazolyl-(1))-3-allyloxybutane,

1-(2-chlorophenyl)-1-(1,2,4-triazolyl-(1))-3-(1-propoxy)-butane,
1-(2-chlorophenyl-1-(1,2,4-triazolyl-(1))-3-(1-butoxy)-butane,
1-(4-chlorophenyl)-1-(1,2,4-triazolyl-(1))-3-ethoxybutane,
1-(4-chlorophenyl)-1-(1,2,4-triazolyl-(1))-3-(1-pentyloxy)-butane,
1-(4-chlorophenyl)-1-(1,2,4-triazolyl-(1))-3-(4-chlorobenzyl)-butane,
1-methoxy-(4-chlorophenyl)-3-(1,2,4-triazolyl-(1))-4,4-dimethylpentane,
1-acetoxy-1-(4-chlorophenyl)-3-(1,2,4-triazolyl-(1))-4,4-dimethylpentane,
1-propionyloxy-1-(4-chlorophenyl)-3-(1,2,4-triazolyl-(1))-4,4-dimethylpentane,
1-acetylacetoxy-1-(4-chlorophenyl)-4-(1,2,4-triazolyl-(1))-4,4-dimethylpentane,
1-acetoxy-1-(4-fluorophenyl)-3-(1,2,4-triazolyl-(1))-4,4-dimethylpentane, and
1-(2,4-dichlorophenyl)-1-(1,2,4-triazolyl-(1))-3-allyloxy-4,4-dimethylpentane.

**Revendications pour les Etats contractants: BE, CH, DE, FR, GB, IT, LU, NL, SE**

1. Composés γ-azolylés de formule générale

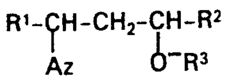

dans laquelle $R^1$ et $R^2$ sont différents ou identiques et représentent alkyle en $C_1$ à $C_8$, furanyle, thiényle, naphtyle ou phényle, le reste phényle pouvant être substitué par fluor, chlore, brome, nitro, trifluorométhyle et, en outre, par alkyle, alcoxy ou alcényle en $C_1$ à $C_4$, et $R^3$ étant un reste alkyle en $C_1$ à $C_8$ ou un reste alcényle en $C_2$ à $C_5$ éventuellement substitué par du chlore ou un reste alcinyle en $C_3$ à $C_4$ ou un reste benzyle pouvant être substitué par fluor, chlore, brome, nitro, trifluorométhyle ou encore par alkyle ou alcoxy en $C_1$ à $C_4$, ou bien $R^3$ représente un reste -CO-$R^4$, $R^4$ étant un reste phényle ou un reste alkyle en $C_1$ à $C_5$ éventuellement substitué par halogène, alcoxy, oxo (= O) ou carboxyalkyle, et $R^3$ représente, en outre, un reste -CO-NH-$R^5$, $R^5$ étant un reste alkyle en $C_1$ à $C_4$ ou un reste chlorophényle, et Az représente un reste imidazolyle, 1,2,4-triazolyle ou 1,2,3-triazolyle lié par l'intermédiaire d'un atome d'azote du noyau.

2. Agent de régularisation de croissance, contenant un composé γ-azolylé selon la revendication 1.

3. Procédé de préparation d'un agent de régularisation de croissance, caractérisé par le fait que l'on mélange un support solide ou liquide avec un composé γ-azolylé selon la revendication 1.

4. Procédé de régularisation de croissance des plantes, caractérisé par le fait que l'on traite les plantes ou le sol avec un composé γ-azolylé selon la revendication 1.

5. Composé γ-azolylé selon la revendication 1 choisi dans le groupe constitué par
1-acétoxy-1-(4-chlorophényl)-3-(1,2,4-triazolyl-(1))-4,4-diméthylpentane,
1-méthoxy-1-phényl-3-(1,2,4-triazolyl-(1))-4,4-diméthylpentane,
1-allyloxy-1-phényl-3-(1,2,4-triazolyl-(1))-4,4-diméthylpentane,
1-méthoxy-1-(4-fluorophényl)-3-(1,2,4-triazolyl-(1))-4,4-diméthylpentane,
1-(2-chlorophényl)-1-(1,2,4-triazolyl-(1))-3-allyloxy-butane,
1-(2-chlorophényl)-1-(1,2,4-triazolyl-(1))-3-(1-propoxy)-butane,
1-(2-chlorophényl)-1-(1,2,4-triazolyl-(1))-3-(1-butoxy)-butane,
1-(4-chlorophényl)-1-(1,2,4-triazolyl-(1))-3-éthoxy-butane,
1-(4-chlorophényl)-1-(1,2,4-triazolyl-(1))-3-(1-pentyloxy)-butane,
1-(4-chlorophényl)-1-(1,2,4-triazolyl-(1))-3-(4-chlorobenzyl)-butane,
1-méthoxy-1-(4-chlorophényl)-3-(1,2,4-triazolyl-(1))-4,4-diméthylpentane.
1-acétoxy-1-(4-chlorophényl)-3-(1,2,4-triazolyl-(1))-4,4-diméthylpentane,
1-propionyloxy-1-(4-chlorophényl)-3-(1,2,4-triazolyl-(1))-4,4-diméthylpentane,
1-acétylacétoxy-1-(4-chlorophényl)-3-(1,2,4-triazolyl-(1))-4,4-diméthylpentane,
1-acétoxy-1-(4-fluorophényl)-3-(1,2,4-triazolyl-(1))-4,4-diméthylpentane,
1-(2,4-dichlorophényl)-1-(1,2,4-triazolyl-(1))-3-allyloxy-4,4-diméthylpentane.

6. Agent de régularisation de croissance, contenant un composé γ-triazolylé selon la revendication 5.

**Revendications pour l'Etat contractant: AT**

1. Agent de régularisation de croissance contenant un composé γ-azolylé de formule générale

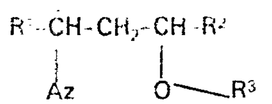

dans laquelle $R^1$ et $R^2$ sont différents ou identiques et représentent alkyle en $C_1$ à $C_8$, furanyle, thiényle, naphtyle ou phényle, le reste phényle pouvant être substitué par fluor, chlore, brome, nitro, trifluorométhyle et, en outre, par alkyle, alcoxy ou alcényle en $C_1$ à $C_4$, et $R^3$ étant un reste alkyle en $C_1$ à $C_8$ ou un reste alcényle en $C_2$ à $C_5$ éventuellement substitué par du chlore ou un reste alcinyle en $C_3$ à $C_4$ ou un reste benzyle pouvant être substitué par fluor, chlore, brome, nitro, trifluorométhyle ou encore par alkyle ou alcoxy en $C_1$ à $C_4$, ou bien $R^3$ représente un reste -CO-$R^4$, $R^4$ étant un reste phényle ou un reste alkyle en $C_1$ à $C_5$ éventuellement substitué par halogène, alcoxy, oxo (= O) ou carboxyalkyle, et $R^3$ représente, en outre, un reste -CO-NH-$R^5$, $R^5$ étant un reste alkyle en $C_1$ à $C_4$ ou un reste chlorophényle, et Az représente un reste imidazolyle, 1,2,4-triazolyle ou 1,2,3-triazolyle lié par l'intermédiaire d'un atome d'azote du noyau.

2. Agent de régularisation de croissance contenant un support solide ou liquide et un composé γ-azolylé selon la revendication 1.

3. Procédé de préparation d'un agent de régularisation de croissance, caractérisé par le fait que l'on mélange un support solide ou liquide avec un composé γ-azolylé selon la revendication 1.

4. Procédé de régularisation de croissance des plantes, caractérisé par le fait que l'on traite les plantes ou le sol avec un composé γ-azolylé selon la revendication 1.

5. Agent de régularisation de la croissance selon la revendication 1 contenant un composé

γ-triazolylé, choisi dans le groupe constitué par
1-acétoxy-1-(4-chlorophényl)-3-(1,2,4-triazolyl-(1))-4,4-diméthylpentane,
1-méthoxy-1-phényl-3-(1,2,4-triazolyl-(1))-4,4-diméthylpentane,
1-allyloxy-1-phényl-3-(1,2,4-triazolyl-(1))-4,4-diméthylpentane,
1-méthoxy-1-(4-fluorophényl)-3-(1,2,4-triazolyl-(1))-4,4-diméthylpentane,
1-(2-chlorophényl)-1-(1,2,4-triazolyl-(1))-3-allyloxy-butane,
1-(2-chlorophényl)-1-(1,2,4-triazolyl-(1))-3-(1-propoxy)-butane,
1-(2-chlorophényl)-1-(1,2,4-triazolyl-(1))-3-(1-butoxy)-butane,
1-(4-chlorophényl)-1-(1,2,4-triazolyl-(1))-3-éthoxy-butane,
1-(4-chlorophényl)-1-(1,2,4-triazolyl-(1))-3-(1-pentyloxy)-butane,
1-(4-chlorophényl)-1-(1,2,4-triazolyl-(1))-3-(4-chlorobenzyl)-butane,
1-méthoxy-1-(4-chlorophényl)-3-(1,2,4-triazolyl-(1))-4,4-diméthylpentane,
1-acétoxy-1-(4-chlorophényl)-3-(1,2,4-triazolyl-(1))-4,4-diméthylpentane,
1-propionyloxy-1-(4-chlorophényl)-3-(1,2,4-triazolyl-(1))-4,4-diméthylpentane,
1-acétylacétoxy-1-(4-chlorophényl)-3-(1,2,4-triazolyl-(1))-4,4-diméthylpentane,
1-acétoxy-1-(4-fluorophényl)-3-(1,2,4-triazolyl-(1))-4,4-diméthylpentane,
1-(2,4-dichlorophényl)-1-(1,2,4-triazolyl-(1))-3-allyloxy-4,4-diméthylpentane.